# EUROPEAN PATENT APPLICATION

(11) **EP 4 368 626 A1**
(43) Date of publication of application: **15.05.2024**
(21) Application number: 23209387.2
(22) Date of filing: 13.11.2023
(51) Int. Cl.: C07F 9/09, C07F 9/12, C07F 9/53, C07F 9/6509, C07F 9/6512

(54) **LIPID COMPOUND OR A DERIVATIVE THEREOF AND PHARMACEUTICAL COMPOSITION EMPLOYING THE SAME**

(30) Priority: 14.11.2022 US 202263425040 P; 23.03.2023 US 202363491843 P; 03.10.2023 TW 112137888
(71) Applicant: Industrial Technology Research Institute, Chutung, Hsinchu 310401 (TW)
(72) Inventor: CHENG, Felice, Hsinchu County (TW); CHENG, Ping-Fu, Hsinchu County (TW); HWANG, Jenn-Tsang, Hsinchu City (TW); FU, Chih-Wei, Taoyuan City (TW); LIN, Ku-Feng, Hsinchu County (TW); CHIU, Ya-Ling, Hsinchu City (TW); LI, Jheng-Sian, Taoyuan City (TW); WANG, Kang-Li, New Taipei City (TW); CHANG, Siou-Han, Tainan City (TW); FAN, Chia-Yu, Hsinchu City (TW)
(74) Representative: Fuchs Patentanwälte Partnerschaft mbB

(57) **Abstract**

A lipid compound or a derivative thereof and a pharmaceutical composition employing the same are provided. The lipid compound has a structure represented by Formula (I): 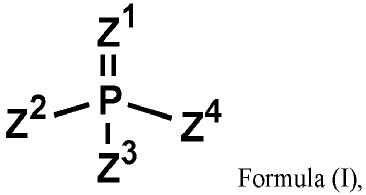 wherein Z¹, Z², Z³ and Z⁴ are as disclosed in the specification.

## Description

### TECHNICAL FIELD

The technical field relates to a lipid compound or a derivative thereof and a pharmaceutical composition employing the same.

### BACKGROUND

The biotechnology industry is prompted to actively invest in the field of nucleic acid drugs due to the success of messenger RNA (mRNA) vaccines, in which lipid nanoparticle (LNP) delivery technology, the key for nucleic acid drugs, is the focus of development. However, ribonucleic acid drugs are easily degraded in the human body and are negatively charged and cannot easily pass through the cell membrane, and thus they need the assistance of carriers to encapsulate them in the carrier for delivery.

Ionizable cationic lipids are currently commonly used carriers for lipid nanoparticles. However, traditional ionizable cationic lipids may result in significant side effects when administered *in vivo.* Observed problems include a low percentage of effective delivery to the target, resulting in relatively low therapeutic effect or low efficacy. Moreover, the ionizable cationic lipid used in the lipid nanoparticles as carriers need to have a specifically tuned pH value so that the carriers can be formulated with the active agent and protect the active agent from degradation during administration, and release the active agent as soon as the carriers reach target thereof.

Accordingly, there is a need in the industry to develop new lipids that can satisfy lipid-nucleic acid delivery systems.

### SUMMARY

According to one embodiment of the present disclosure, the present disclosure provides a lipid compound or a derivative thereof, wherein the derivative of the lipid compound may be a pharmaceutically acceptable salt of the lipid compound or a solvate of the lipid compound. According to one embodiment of the present disclosure, the lipid compound has a structure represented by Formula (I): 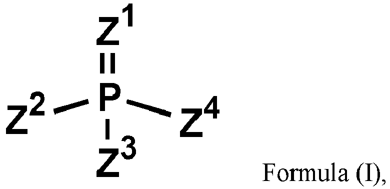 wherein Z¹ is O or S; Z² is -Q¹-A¹-Q²-A²-X¹; Z³ and Z² are the same; Z⁴ is -Q³-A³-Q⁴-A⁴-X²; each of Q¹ and Q³ is independently a single bond, -O- or -NH-; each of Q² and Q⁴ is independently a single bond, -O-, -NH-, -S-S-, 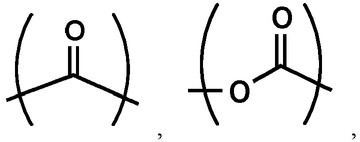 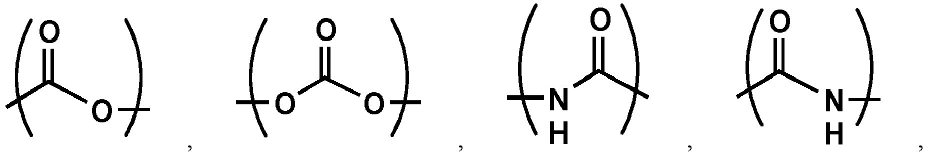 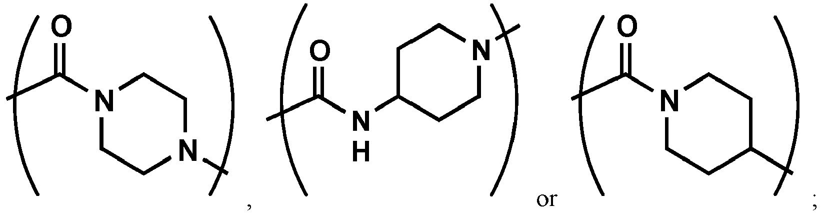 A¹ is C1-C6 alkylene group; A³ is a single bond or a C1-C6 alkylene group; each of A² and A⁴ is independently a single bond, a C1-C12 alkylene group, a C2-C12 alkenylene group or a C2-C12 alkynylene group; X¹ is hydrogen, a C1-C24 alkyl group, a C2-C24 alkenyl group, a C2-C24 alkynyl group, a C1-C24 alkanol group, a C2-C24 alkenol group, a C2-C24 alkynol group, -NR¹R², 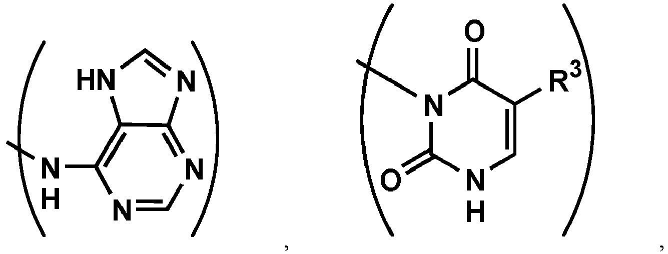 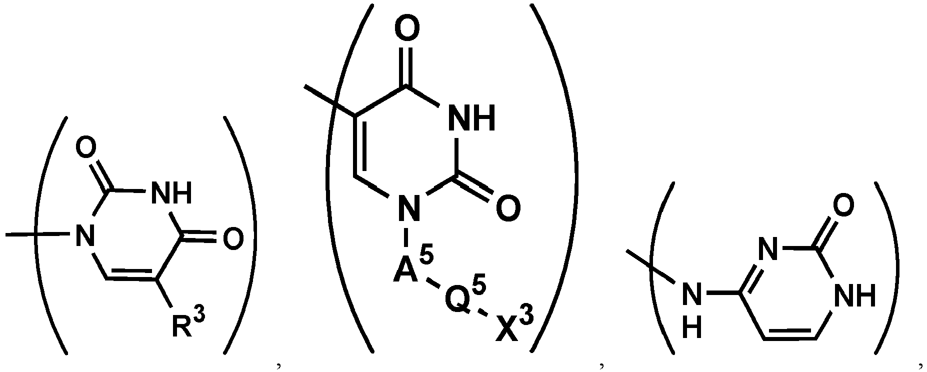 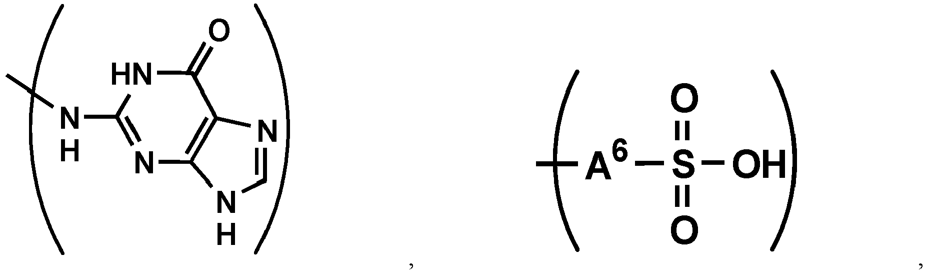 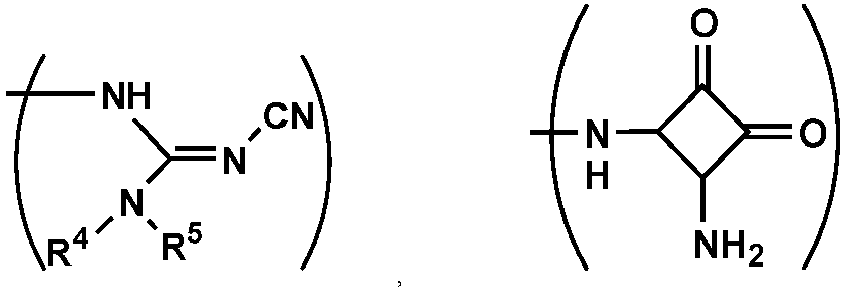 or 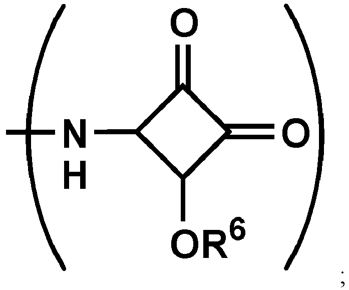 X² is hydrogen, a C6-C12 aryl group, a C1-C24 alkyl group, a C2-C24 alkenyl group, a C2-C24 alkynyl group, a C1-C24 alkanol group, a C2-C24 alkenol group, a C2-C24 alkynol group, -NR⁷R⁸, 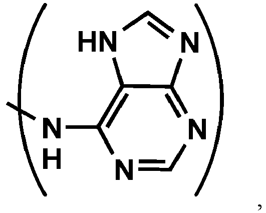 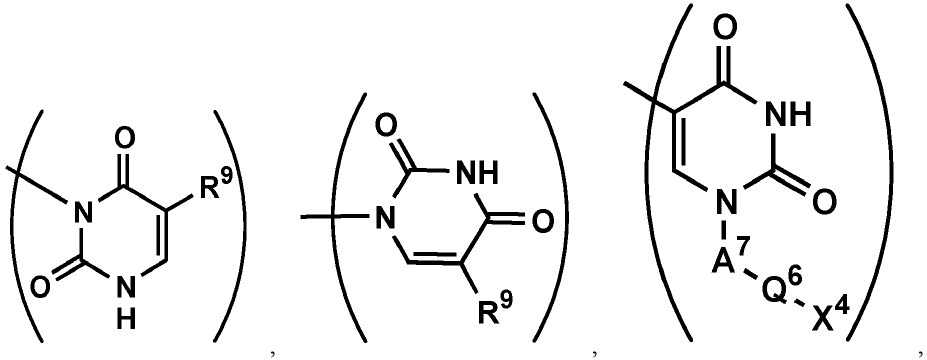 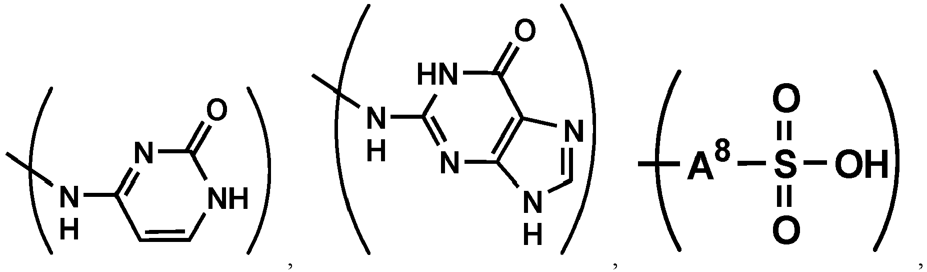 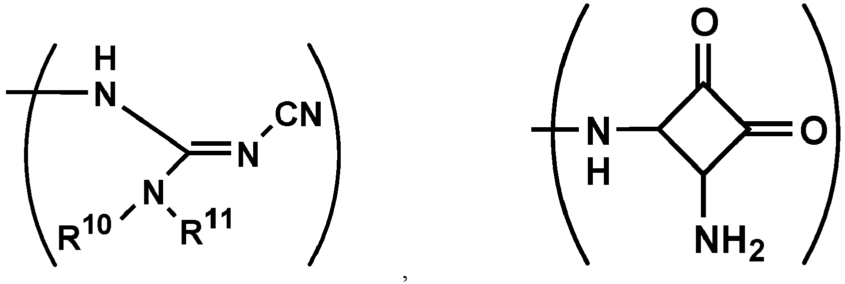 or 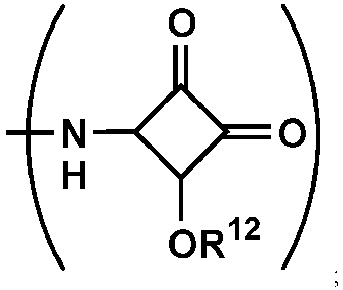 R¹ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A⁹-Q⁷-X⁵; R² is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹⁰-Q⁸-X⁶; each of R³, R⁴, R⁵, R⁶, R⁹, R¹⁰, R¹¹ and R¹² is independently hydrogen or a C1-C6 alkyl group; R⁷ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹¹-Q⁹-X⁷; R⁸ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹²-Q¹⁰-X⁸; each of A⁵, A⁶, A⁷, A⁸, A⁹, A¹⁰, A¹¹ and A¹² is independently a C1-C12 alkylene group; each of Q⁵, Q ⁶, Q ⁷, Q ⁸, Q ⁹ and Q ¹⁰ is independently -O-, -NH-, -S-S-, 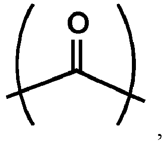 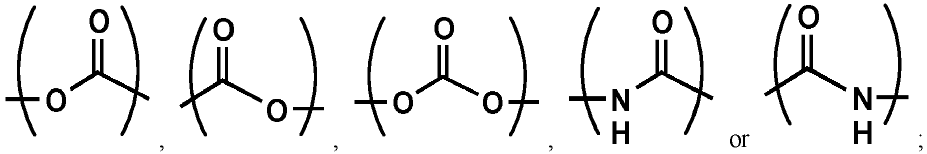 and each of X³, X⁴, X⁵, X⁶, X⁷ and X⁸ is independently a C1-C24 alkyl group.

According to some embodiments of the present disclosure, the present disclosure provides a pharmaceutical composition, wherein the pharmaceutical composition comprises the lipid compound or a derivative thereof mentioned by the present disclosure and a helper lipid.

A detailed description is given in the following embodiments with reference to the accompanying drawings.

### BRIEF DESCRIPTION OF DRAWINGS

The present invention can be more fully understood by reading the subsequent detailed description and examples with references made to the accompanying drawings, wherein:

None.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The following is directed to the lipid compound mentioned in the present disclosure or a derivative thereof and a pharmaceutical composition employing the same. It should be understood that the following description provides many different embodiments or examples for implementing the present disclosure. The specific components and arrangements described below are merely descriptive of the present disclosure. Of course, these are only examples and not limitations of the present disclosure. In present disclosure, the word "about" means that the specified amount increases or decreases an amount that is generally recognized as reasonable by a person skilled in the art.

The present disclosure provides a lipid compound or a derivative thereof. According to one embodiment of the present disclosure, the lipid compound mentioned in the present disclosure is an ionizable cationic phospholipid with a specific structure of which the main core is pentavalent phosphorus and which may include 1 to 3 ionizable tertiary amine groups. The lipid compound mentioned in the present disclosure is easier to metabolize *in vivo* and has lower toxicity. The lipid compound or a derivative thereof mentioned in the present disclosure can be positively charged in an acidic environment, thereby adsorbing a negatively charged nucleic acid to form lipid nanoparticles (LNP). In addition, the present disclosure also provides a pharmaceutical composition comprising the lipid compound or a derivative thereof mentioned in the present disclosure. By having the lipid compound or a derivative thereof mentioned in the present disclosure, the pharmaceutical composition mentioned in the present disclosure can form lipid nanoparticles (LNPs) encapsulating ribonucleic acid (RNA) and/or deoxyribonucleic acid (DNA), thereby achieving the purposes such as protection of ribonucleic acid (RNA) and/or deoxyribonucleic acid (DNA), slowing down its degradation rate, and improving cell transfection efficiency.

According to one embodiment of the present disclosure, the lipid compound provided by the present disclosure has a structure represented by Formula (I): 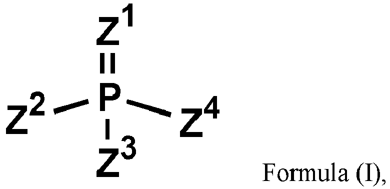 wherein Z¹ may be O or S; Z² may be -Q¹-A¹-Q²-A²-X¹; Z³ and Z² are the same; Z⁴ may be -Q³-A³-Q⁴-A⁴-X²; each of Q¹ and Q³ may be independently a single bond, -O- or -NH-; each of Q² and Q⁴ may be independently a single bond, -O-, -NH-, -S-S-, 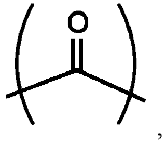 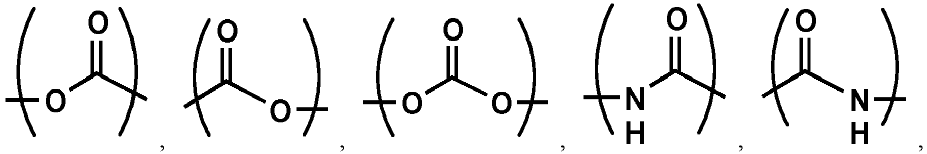 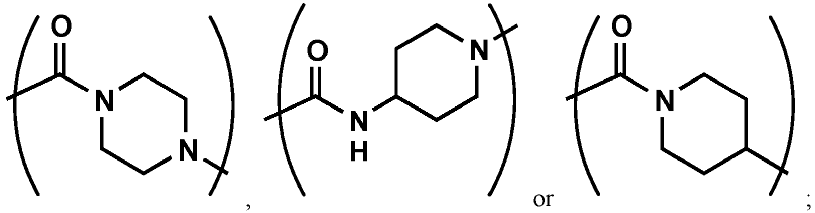 A¹ may be C1-C6 alkylene group; A³ may be a single bond or a C1-C6 alkylene group; each of A² and A⁴ may be independently a single bond, a C1-C12 alkylene group, a C2-C12 alkenylene group or a C2-C12 alkynylene group; X¹ may be hydrogen, a C1-C24 alkyl group, a C2-C24 alkenyl group, a C2-C24 alkynyl group, a C1-C24 alkanol group, a C2-C24 alkenol group, a C2-C24 alkynol group, -NR¹R², 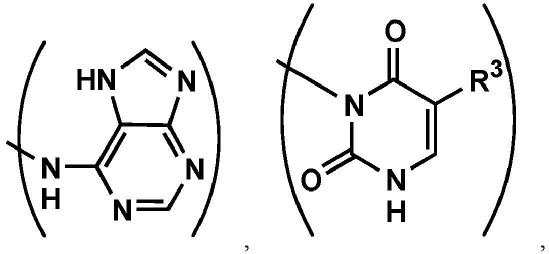 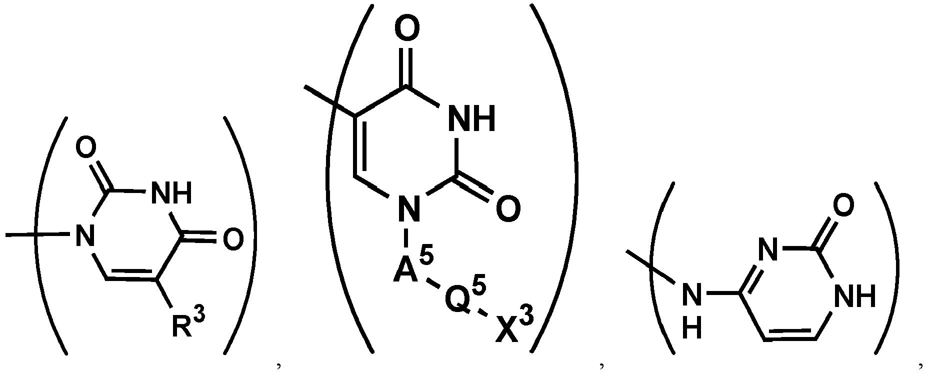 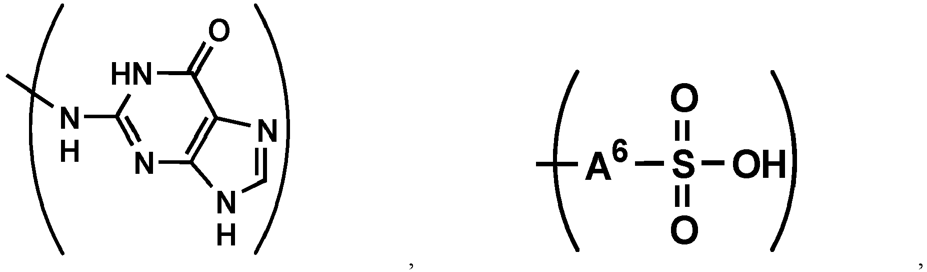 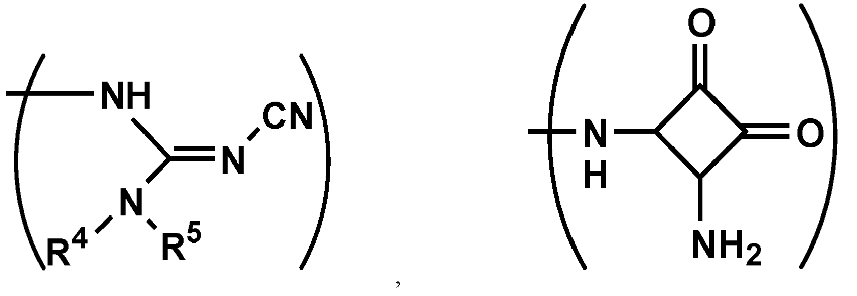 or 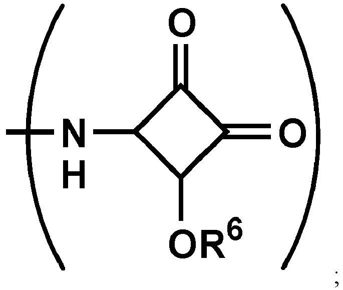 X² may be hydrogen, a C6-C12 aryl group, a C1-C24 alkyl group, a C2-C24 alkenyl group, a C2-C24 alkynyl group, a C1-C24 alkanol group, a C2-C24 alkenol group, a C2-C24 alkynol group, -NR⁷R⁸, 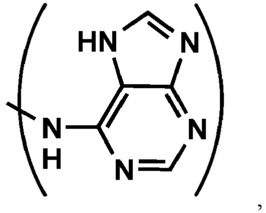 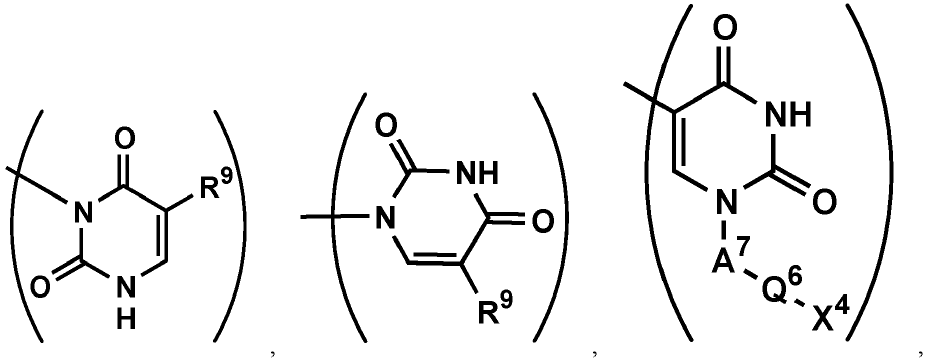 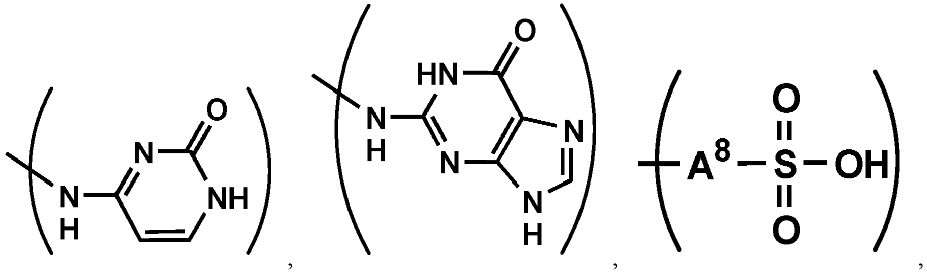 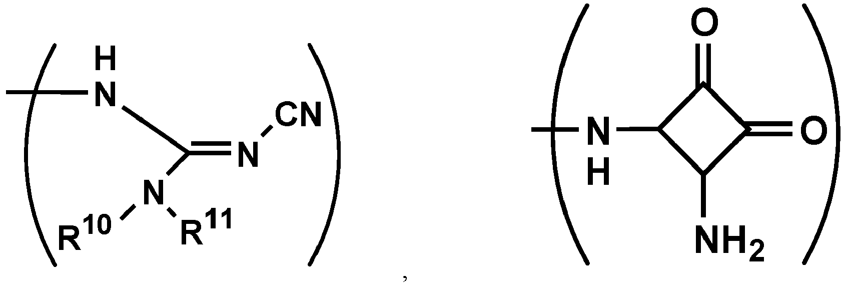 or 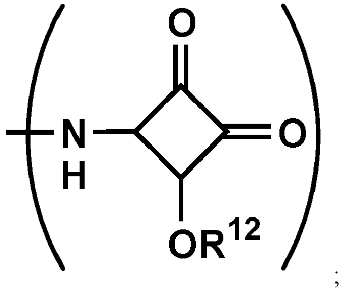 R¹ may be hydrogen, a C1-C24 alkyl group, a C2-C24 alkenyl group, a C2-C24 alkynyl group, a C1-C24 alkanol group, a C2-C24 alkenol group, a C2-C24 alkynol group or -A⁹-Q⁷-X⁵; R² may be hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹⁰-Q⁸-X⁶; each of R³, R⁴, R⁵, R⁶, R⁹, R¹⁰, R¹¹ and R¹² may be independently hydrogen or a C1-C6 alkyl group; R⁷ may be hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹¹-Q⁹-X⁷; R⁸ may be hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹²-Q¹⁰-X⁸; each of A⁵, A⁶, A⁷, A⁸, A⁹, A¹⁰, A¹¹ and A¹² may be independently a C1-C12 alkylene group; each of Q⁵, Q⁶, Q⁷, Q⁸, Q⁹ and Q ¹⁰ may be independently -O-, -NH-, -S-S- 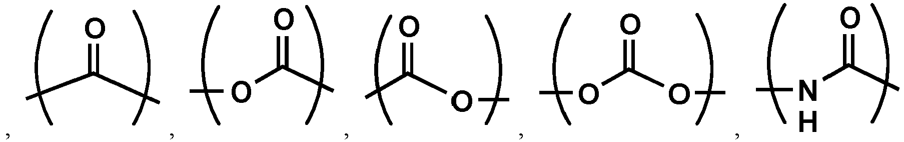 or 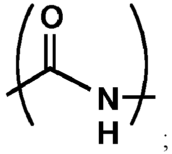 and each of X³, X⁴, X⁵, X⁶, X⁷ and X⁸ may be independently a C1-C24 alkyl group.

According to one embodiment of the present disclosure, the derivative of the lipid compound mentioned in the present disclosure may be a pharmaceutically acceptable salt of the lipid compound or a solvate of the lipid compound.

According to one embodiment of the present disclosure, the pharmaceutically acceptable salt of the lipid compound may be formed by reacting the lipid compound with a pharmaceutically acceptable acid. Here, the pharmaceutically acceptable salt includes a salt formed by the lipid compound and an inorganic acid, such as a hydrochloride, a phosphate, a diphosphate, a hydrobromide, a sulfate, a sulfinate, or a nitrate. According to one embodiment of the present disclosure, the pharmaceutically acceptable salt includes a salt formed by the lipid compound and an organic acid, such as lactate, oxalate, malate, maleate, fumarate, tartrate, succinate, citrate, lactate, sulfonate, p-toluenesulfonate, 2-hydroxyethanesulfonate, benzoate, salicylate, stearate, trifluoroacetate, amino acid salt, or acetate. In addition, pharmaceutically acceptable cations include, but are not limited to, sodium, potassium, calcium, aluminum, lithium and ammonium.

According to one embodiment of the present disclosure, the solvate of the lipid compound includes the hydrate of the lipid compound or the alcoholate of the lipid compound.

According to one embodiment of the present disclosure, a single bond mentioned in the present disclosure refers to the absence of a single atom at the relevant site. For example, in the structure where Z² is -Q¹-A¹-Q²-A²-X¹, when Q² is a single bond, there are no individual atoms at the site represented by Q², and A¹ and A² are directly connected such that Z² is a structure of -Q¹-A¹-A²-X¹.

According to one embodiment of the present disclosure, the alkyl group mentioned in the present disclosure may be a linear or branched alkyl group. According to one embodiment of the present disclosure, the alkenyl group mentioned in the present disclosure may be a linear or branched alkenyl group and include at least one carbon-carbon double bond. According to one embodiment of the present disclosure, the alkynyl group mentioned in the present disclosure may be a linear or branched alkynyl group and include at least one carbon-carbon triple bond).

For example, C1-C24 alkyl may be methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl or an isomer thereof.

According to one embodiment of the present disclosure, the alkanol group mentioned in the present disclosure may be a linear or branched alkanol group. According to one embodiment of the present disclosure, the alkenol group mentioned in the present disclosure may be a linear or branched alkenol group and include at least one carbon-carbon double bond. According to one embodiment of the present disclosure, the alkynol group mentioned in the present disclosure may be a linear or branched alkynyl group and include at least one carbon-carbon triple bond. Herein, the term "alkanol group" refers to an alkyl group in which at least one of its hydrogen on carbon is replaced by a hydroxyl group. The term "alkenol group" refers to an alkenyl group in which at least one of its hydrogen on carbon is replaced by a hydroxyl group. The term "alkynol group" refers to an alkynyl group in which at least one of its hydrogen on carbon is replaced by a hydroxyl group. In addition, According to one embodiment of the present disclosure, the C6-C12 aryl group mentioned in the present disclosure may be phenyl, biphenyl or naphthyl.

According to one embodiment of the present disclosure, the alkylene group mentioned in the present disclosure may be a linear or branched alkylene group. According to one embodiment of the present disclosure, the alkenylene group mentioned in the present disclosure may be a linear or branched alkenylene group. According to one embodiment of the present disclosure, the alkynylene group mentioned in the present disclosure may be a linear or branched alkynylene group.

For example, C1-C24 alkylene group may be methylene group, ethylene group, propylene group, butylene group, pentylene group, hexylene group or an isomer thereof.

According to one embodiment of the present disclosure, for the lipid compound having a structure represented by Formula (I) mentioned in the present disclosure, Z⁴ is different from Z² (i.e. Z⁴ is different from Z³).

According to one embodiment of the present disclosure, for the lipid compound having a structure represented by Formula (I) mentioned in the present disclosure, Z², Z³ and Z⁴ are the same.

According to one embodiment of the present disclosure, for the lipid compound having a structure represented by Formula (I) mentioned in the present disclosure, Q¹ and Q² will not be single bonds at the same time. In other words, when Q¹ is a single bond, Q² is not a single bond; and when Q² is a single bond, Q¹ is not a single bond.

According to one embodiment of the present disclosure, Q¹ is a single bond, and Q² may be -O-, -NH-, -S-S-,

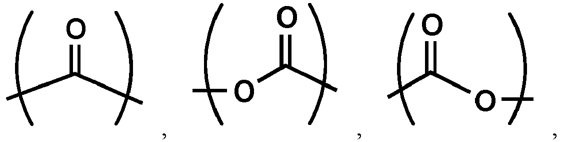

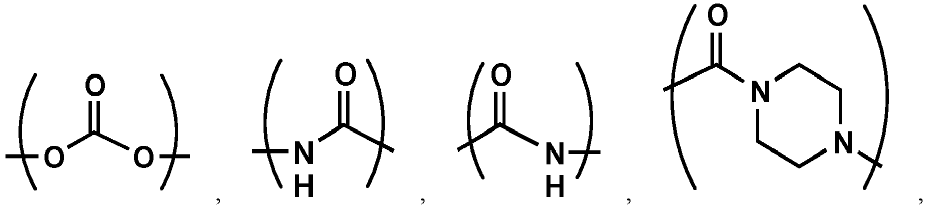

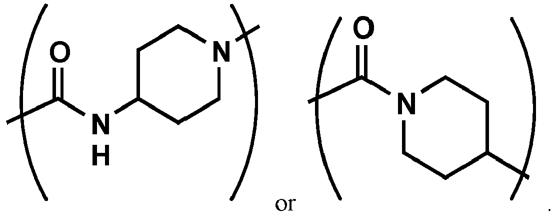

According to one embodiment of the present disclosure, Q² is a single bond, and Q¹ may be -O- or -NH-.

According to one embodiment of the present disclosure, Q¹ may be -O- or -NH-, and Q² may be -O-, -NH-, -S-S-,

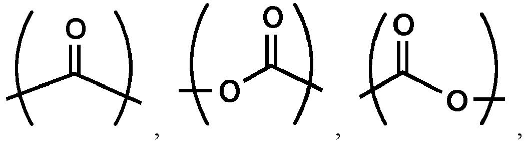

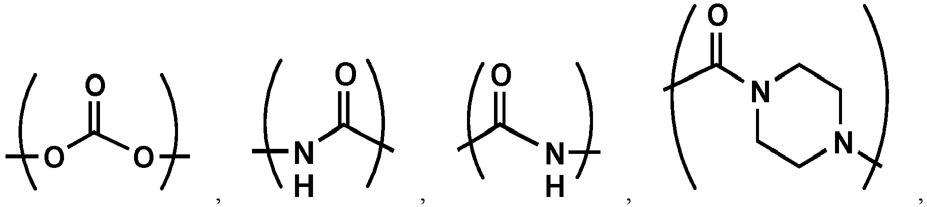

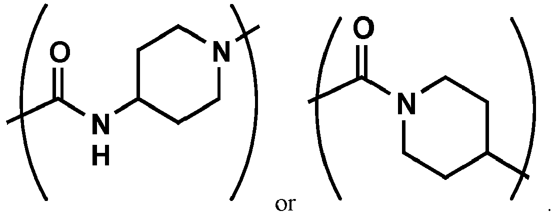

According to one embodiment of the present disclosure, for the lipid compound having a structure represented by Formula (I) mentioned in the present disclosure, Q³ and Q⁴ will not be single bonds at the same time. In other words, when Q³ is a single bond, Q⁴ is not a single bond; and when Q⁴ is a single bond, Q³ is not a single bond.

According to one embodiment of the present disclosure, Q³ is a single bond, and Q⁴ may be -O-, -NH-, -S-S-,

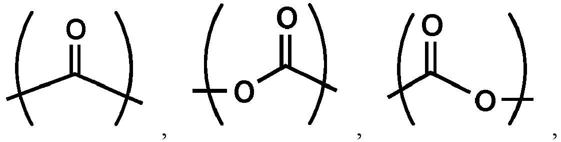

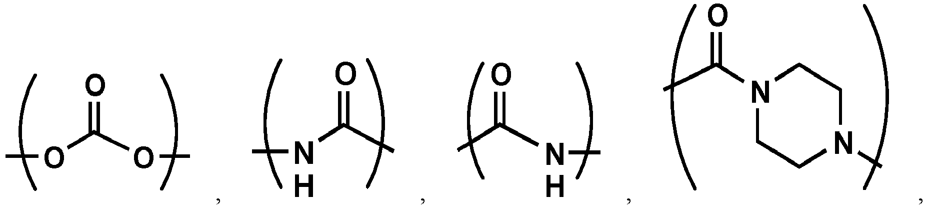

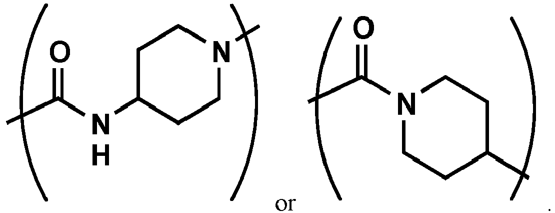

According to one embodiment of the present disclosure, Q⁴ is a single bond, and Q³ may be -O- or -NH-.

According to one embodiment of the present disclosure, Q³ may be -O- or -NH-, Q⁴ may be -O-, -NH-, -S-S-, 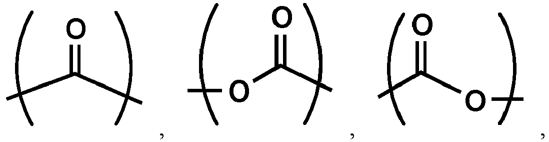 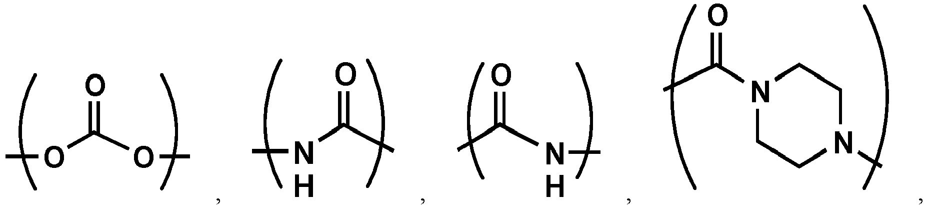 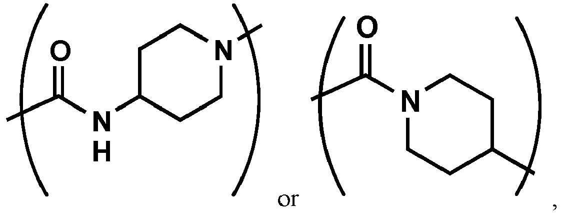 and A³ is not a single bond.

According to one embodiment of the present disclosure, when A² is a C1-C12 alkylene group, a C2-C12 alkenylene group or a C2-C12 alkynylene group, Q² is not a single bond.

According to one embodiment of the present disclosure, when A³ is a single bond, A⁴ is a C1-C12 alkylene group, a C2-C12 alkenylene group or a C2-C12 alkynylene group.

According to one embodiment of the present disclosure, when A⁴ is a single bond, A³ is a C1-C6 alkylene group.

According to one embodiment of the present disclosure, when A³ is a single bond and A⁴ is a single bond, Q³ is -O- or -NH-.

According to one embodiment of the present disclosure, the lipid compound having a structure represented by Formula (I) mentioned in the present disclosure may be 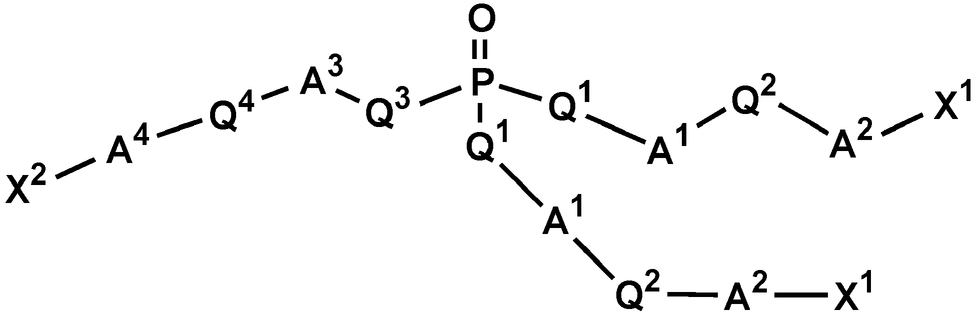 or 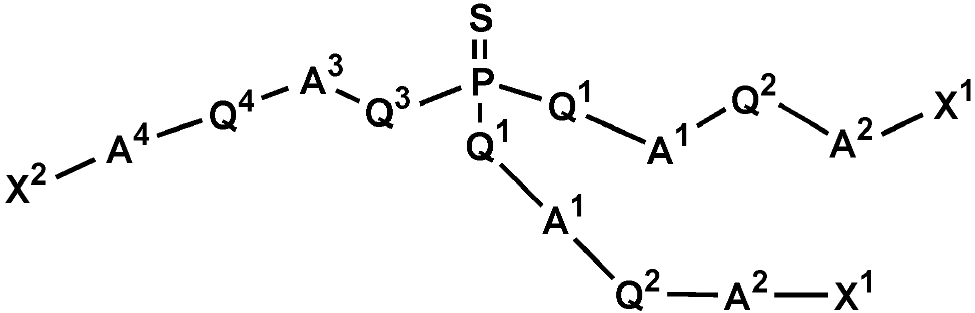 , wherein the definitions of Q¹, Q², Q³, Q⁴, A¹, A², A³, A⁴, X¹ and X² are the same as above.

According to one embodiment of the present disclosure, the present disclosure also provide a pharmaceutical composition, such as a lipid nanoparticle composition. According to one embodiment of the present disclosure, the pharmaceutical composition comprise the lipid compound or a derivative thereof mentioned in the present disclosure and a sterol.

According to one embodiment of the present disclosure, in the pharmaceutical composition, the lipid compound (and/or a derivative thereof) is 100 parts by mole, and the content of the sterol may be 50 to 300 parts by mole, such as 60 parts by mole, 80 parts by mole, 100 parts by mole, 120 parts by mole, 150 parts by mole, 180 parts by mole, 200 parts by mole, 230 parts by mole, 250 parts by mole or 280 parts by mole. According to one embodiment of the present disclosure, the sterol may be cholesterol, cholesterol hexasuccinate, ergosterol, lanosterol, or a combination thereof.

According to one embodiment of the present disclosure, the pharmaceutical composition may further comprise a helper lipid, wherein the helper lipid may be 5 to 200 parts by mole, such as 6 parts by mole, 8 parts by mole, 10 parts by mole, 20 parts by mole, 30 parts by mole, 50 parts by mole, 75 parts by mole, 100 parts by mole, 120 parts by mole, 150 parts by mole, 170 parts by mole or 190 parts by mole.

According to one embodiment of the present disclosure, the helper lipid may be 1, 2-dilauroyl-sn-glycero-3-phosphocholine (DLPC), 1, 2-dimyristoyl-sn-glycero-3-phosphocholine (DMPC), 1, 2-dipalmitoyl-sn-glycero-3-phosphocholine (DPPC), 1-palmitoyl-2-stearoyl-sn-glycero-3-phosphocholine (PSPC), 1-palmitoyl 2-oleoyl-sn-glycero-3-phosphatidylcholine (POPC), 1, 2-distearoyl-sn-glycero-3-phosphocholine (DSPC), 1, 2-dioleoyl-sn-glycero-3-phosphocholine (DOPC), hydrogenated soy phosphatidylcholine (HSPC), 1-stearoyl-2-oleoyl-sn-glycero-3-phosphocholine (SOPC), 1, 2-dimyristoyl-sn-glycero-3-phospho-(1'-rac-glycerol)(sodium salt)(DMPG), sulfoquinovosyl diacylglycerol (SQDG), monogalactosyldiacylglycerol (MGDG), digalactosyldiacylglycerol (DGDG), 1, 2-dioleoyl-sn-glycero-3-phosphoethanolamine (DOPE), 1-palmitoyl-2-oleoyl-sn-glycero-3-phosphoethanolamine (POPE), 1, 2-dioleoyl-sn-glycero-3-phospho-1-serine (DOPS), L-a-phosphatidylcholine (EPC), phosphatidylcholine (PC) or a combination thereof.

According to one embodiment of the present disclosure, the pharmaceutical composition may further comprise a polyethylene glycol (PEG)-ylated lipid, wherein the content of the PEGylated lipid may be 1 to 30 parts by mole, such as 2 parts by mole, 3 parts by mole, 4 parts by mole, 5 parts by mole, 6 parts by mole, 7 parts by mole, 8 parts by mole, 9 parts by mole, 10 parts by mole, 11 parts by mole, 12 parts by mole, 13 parts by mole, 14 parts by mole, 15 parts by mole, 16 parts by mole, 17 parts by mole, 18 parts by mole, 19 parts by mole, 20 parts by mole, 21 parts by mole, 22 parts by mole, 23 parts by mole, 24 parts by mole, 25 parts by mole, 26 parts by mole, 27 parts by mole, 28 parts by mole or 29 parts by mole.

According to one embodiment of the present disclosure, the PEGylated lipid may be a polyethylene glycol-modified phosphatidylethanolamine, a polyethylene glycol-modified phosphatidic acid, a polyethylene glycol-modified ceramide, a polyethylene glycol-modified dialkyl amine, a polyethylene glycol-modified diacylglycerol, a polyethylene glycol-modified dialkyl glycerol, a polyethylene glycol-modified sterol, a polyethylene glycol-modified phospholipid or a combination thereof.

According to one embodiment of the present disclosure, the PEGylated lipid may be 1, 2-dimyristoyl-sn-glycero-3-phosphoethanolamine-polyethylene glycol (DMPE-PEG), 1, 2-distearoyl-sn-glycero-3-phosphoethanolamine-polyethylene glycol (DSPE-PEG), 1, 2-dimyristoyl-sn-glycero-3-phosphoethanolamine-polyethylene glycol (DMPE-PEG), 1, 2-Dipalmitoyl-sn-glycerol-3-succinate-polyethylene glycol (DPGS-PEG), cholesteryl-polyethylene glycol, 1, 2-dipalmitoyl-sn-glycero-3-phosphoethanolamine-polyethylene glycol (DPPE-PEG), 1, 2-dioleoyl-sn-glycero-3-phosphoethanolamine-polyethylene glycol (DOPE-PEG), 1, 2-dimyristoyl-sn-glycerol-methoxypolyethylene glycol (DMG-PEG), 1, 2-distearoyl-sn-glycerol-3-methoxy-polyethylene glycol (DSG-PEG), 1, 2-dipalmitoyl-sn-glycerol-methoxy-polyethylene glycol (DPG-PEG), α-[2-(ditetradecylamino)-2-oxoethyl]-ω-methoxy-poly(oxy-1,2-ethanediyl) (ALC-0159) or a combination thereof.

According to one embodiment of the present disclosure, the pharmaceutical composition may further comprise a nucleic acid, wherein the content of the nucleic acid may be 0.1 to 30 parts by mole, such as 0.2 parts by mole, 0.5 parts by mole, 1 molar part, 1.5 parts by mole, 2 parts by mole, 3 parts by mole, 4 parts by mole, 5 parts by mole, 6 parts by mole, 7 parts by mole, 8 parts by mole, 9 parts by mole, 10 parts by mole, 11 parts by mole, 12 parts by mole, 13 parts by mole, 14 parts by mole, 15 parts by mole, 16 parts by mole, 17 moles parts by mole, 18 parts by mole, 19 parts by mole, 20 parts by mole, 21 parts by mole, 22 parts by mole, 23 parts by mole, 24 parts by mole, 25 parts by mole, 26 parts by mole, 27 parts by mole, 28 parts by mole or 29 parts by mole.

According to one embodiment of the present disclosure, the nucleic acid may be deoxyribonucleic acid (DNA), plasmid DNA, messenger ribonucleic acid (mRNA), small interfering ribonucleic acid (siRNA), small activated ribonucleic acid (saRNA), circular ribonucleic acid (circular RNA) or a combination thereof.

In order to make the above content and other objects, features, and advantages of the present disclosure more obvious and understandable, exemplary examples are shown below and described in detail, but the present disclosure is not limited thereto.

### Lipid compound

The lipid compounds mentioned in Examples of the present disclosure are listed in Table 1.

**Table 1**

| | Structure |
|---|---|
| Example 1 | 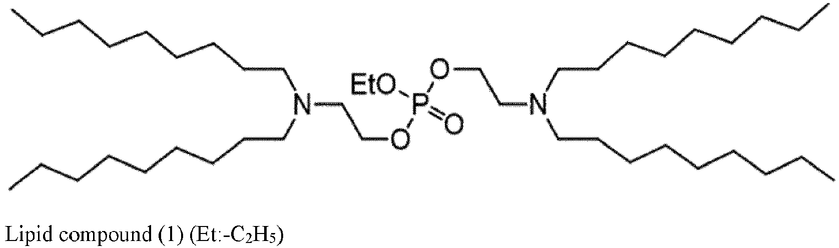 |
| Example 2 | 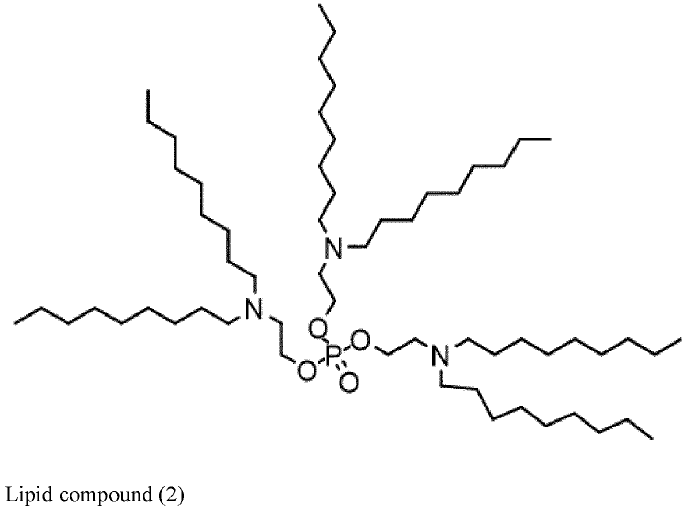 |
| Example 3 | 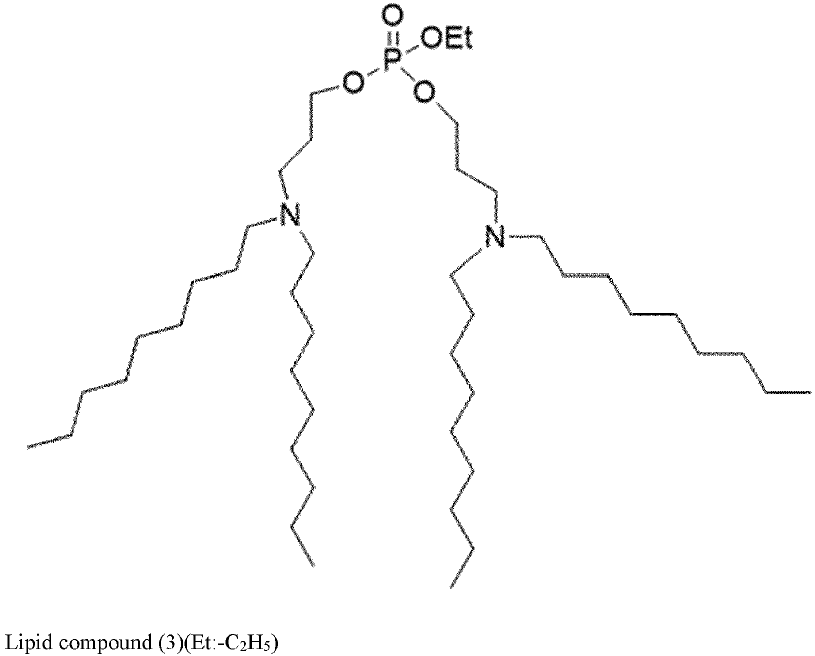 |
| Example 4 | 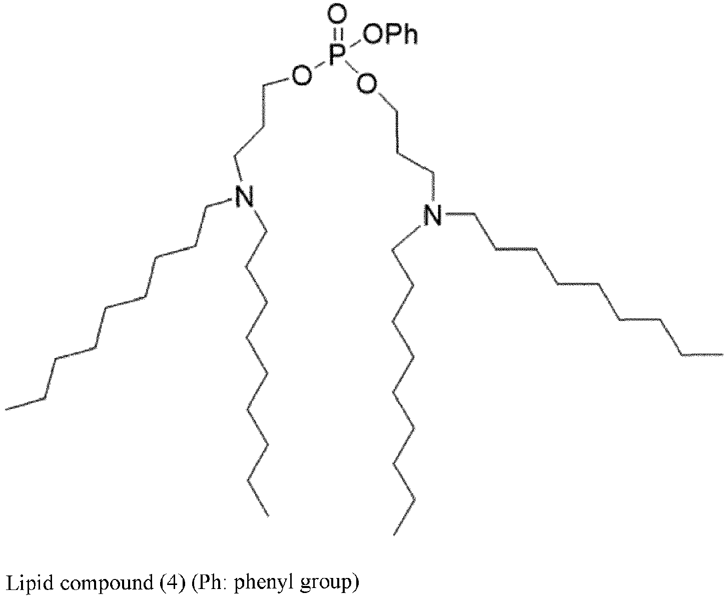 |
| Example 5 | 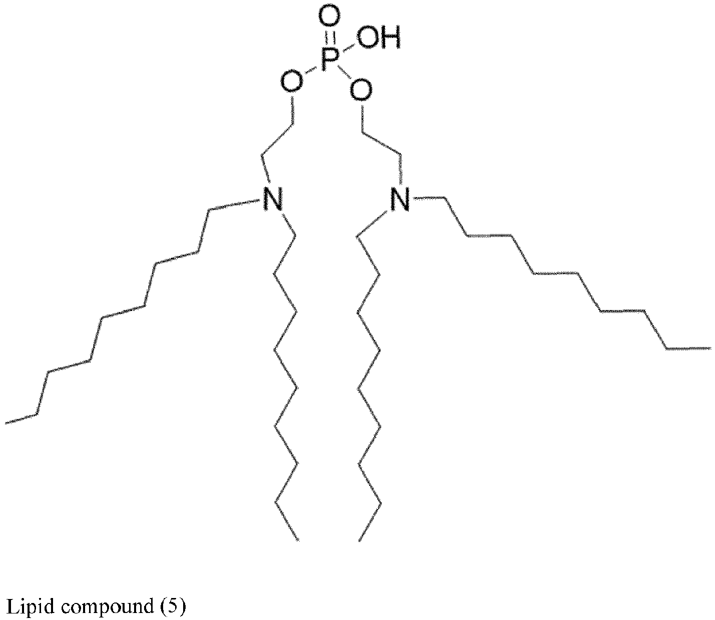 |
| Example 6 | 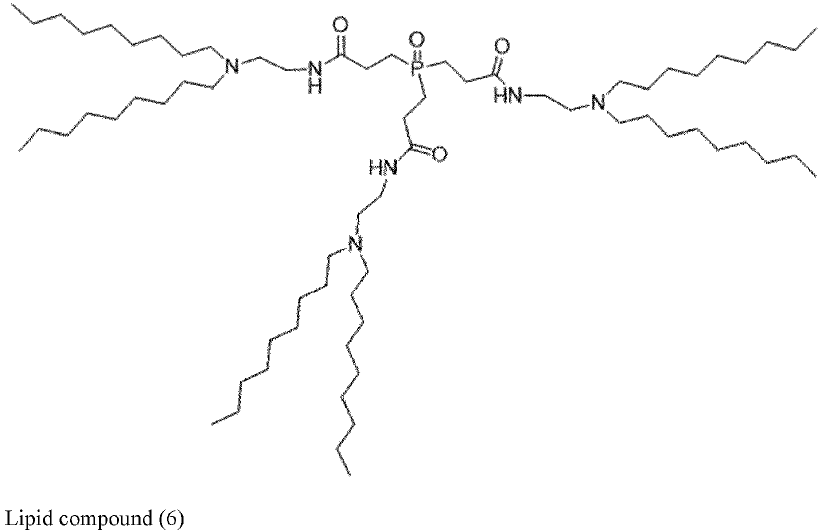 |
| Example 7 | 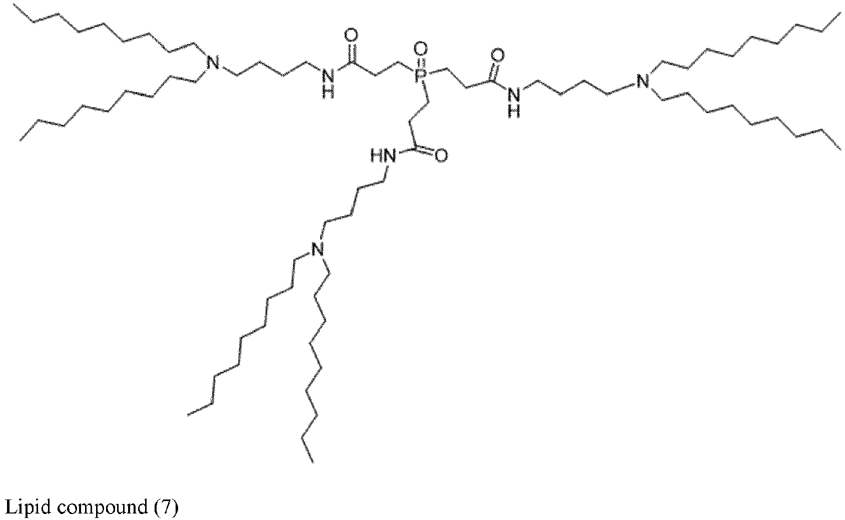 |
| Example 8 | 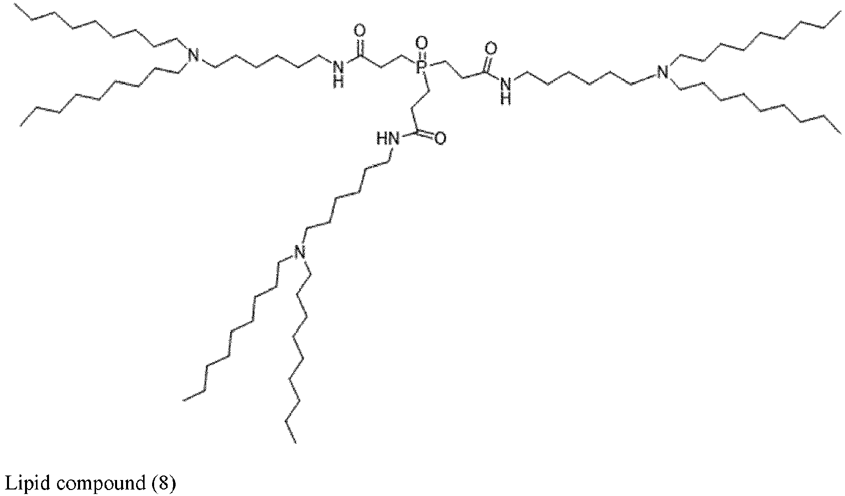 |
| Example 9 | 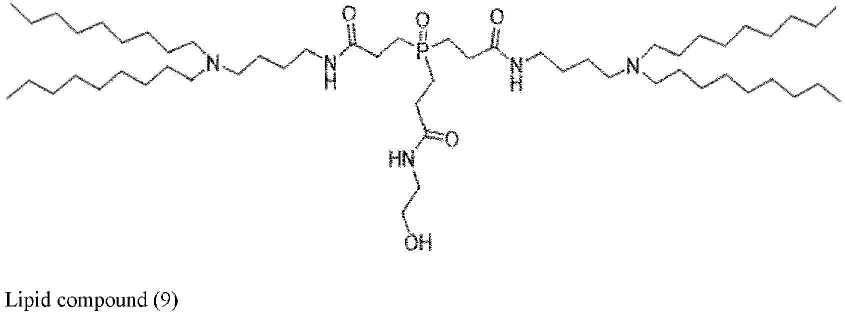 |
| Example 10 | 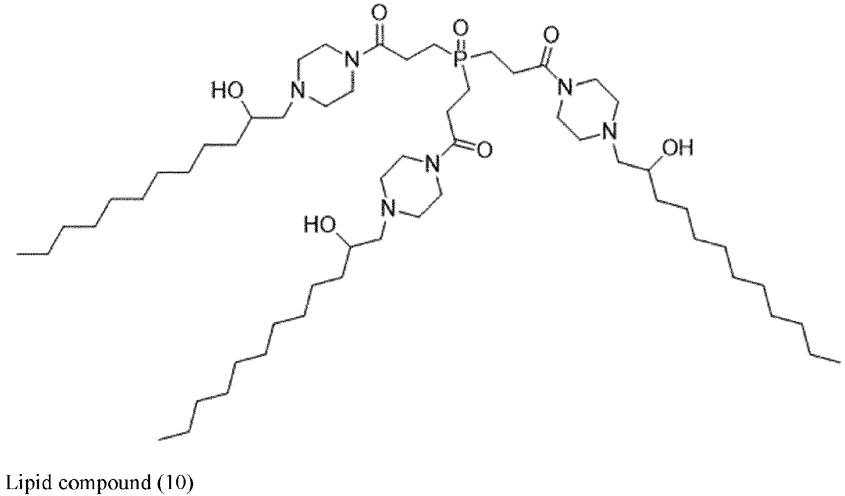 |
| Example 11 | 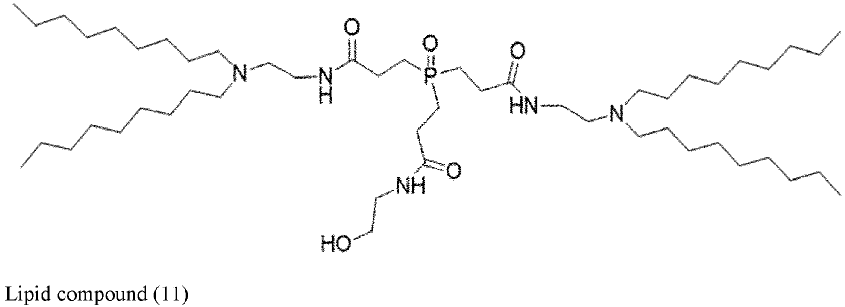 |
| Example 12 | 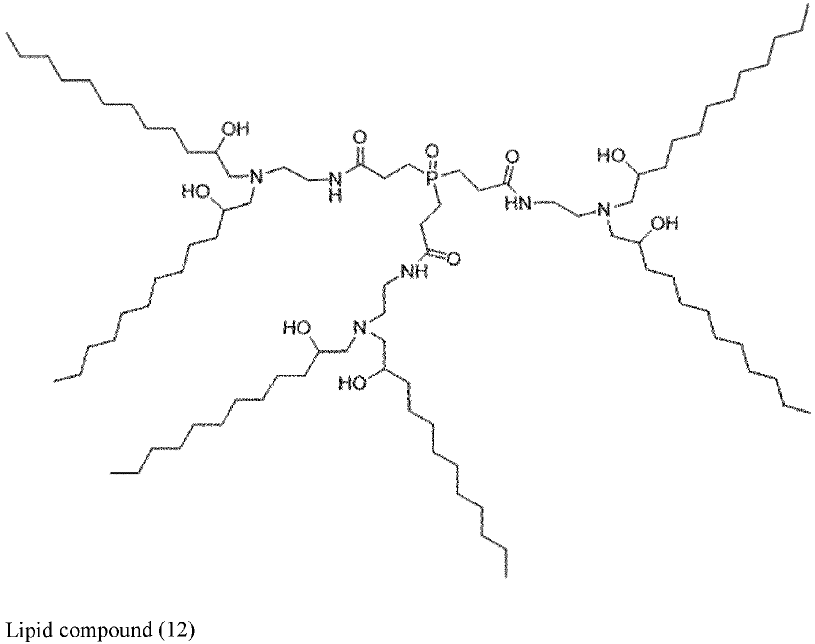 |
| Example 13 | 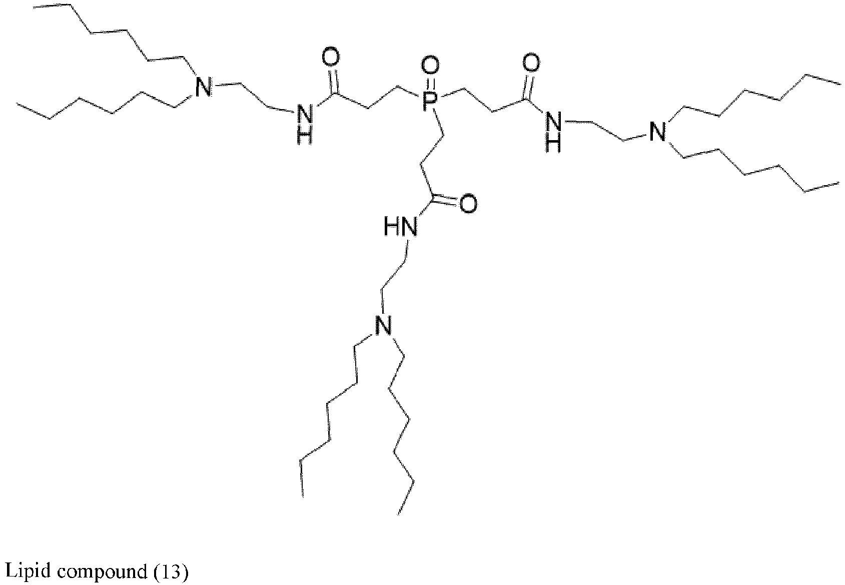 |
| Example 14 | 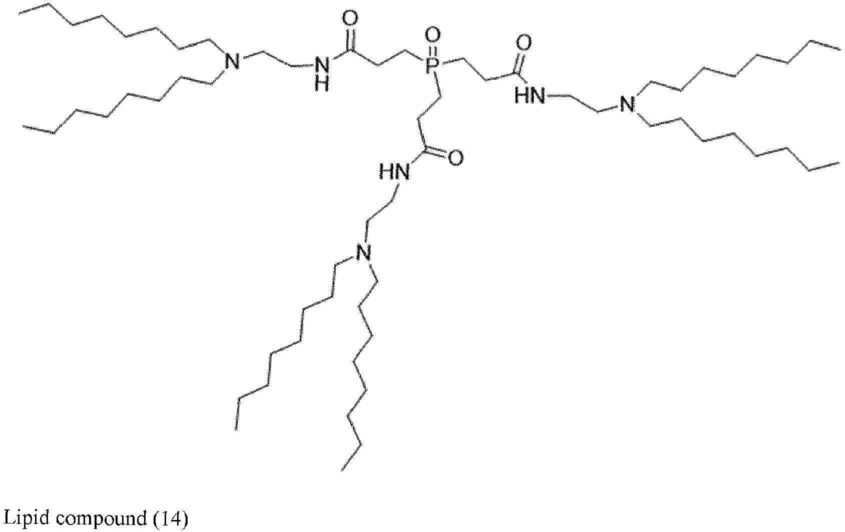 |
| Example 15 | 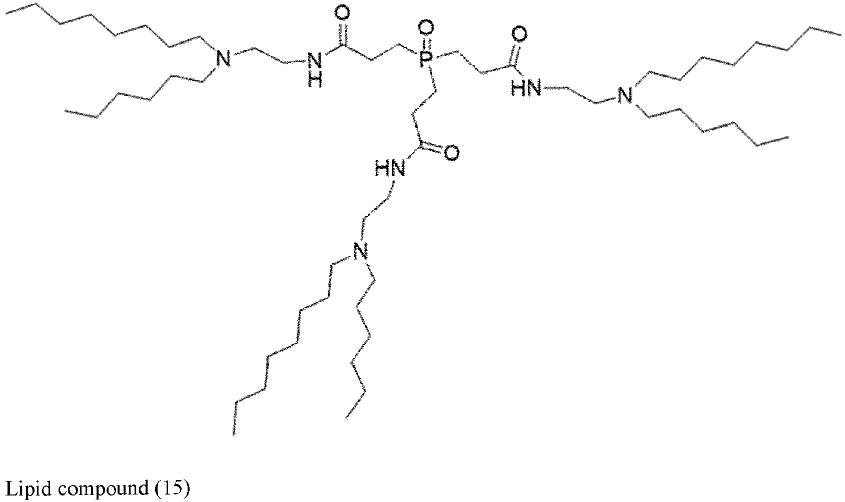 |
| Example 16 | 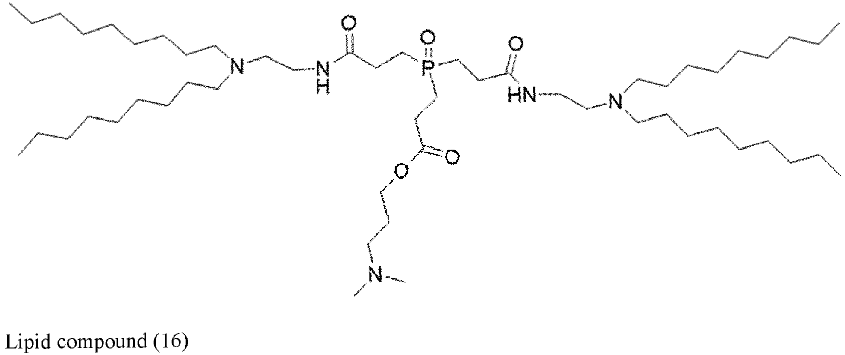 |
| Example 17 | 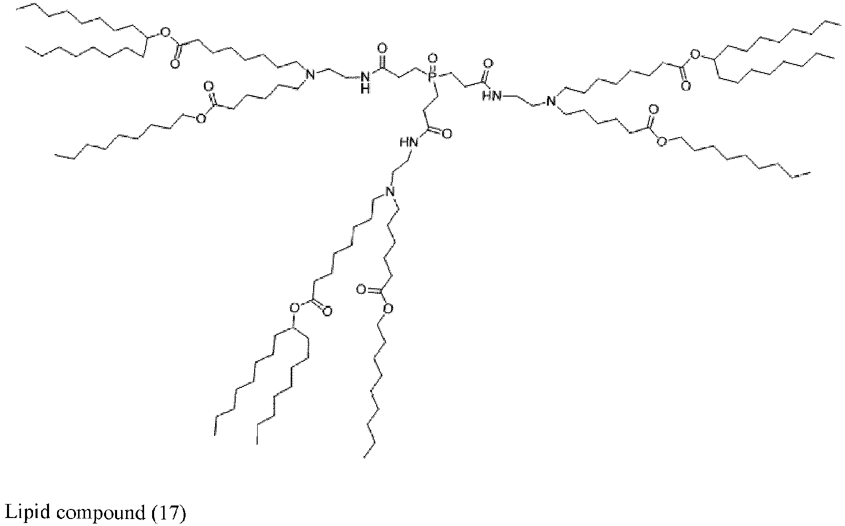 |
| Example 18 | 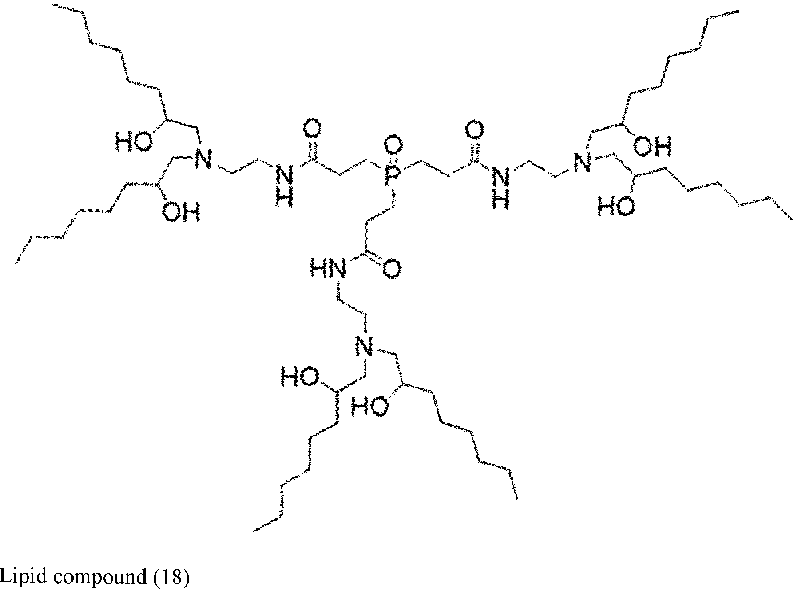 |
| Example 19 | 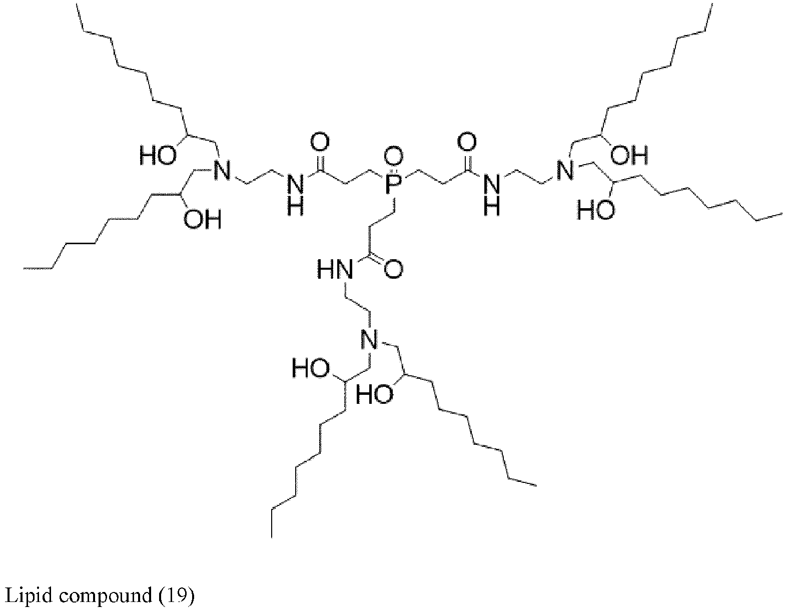 |
| Example 20 | 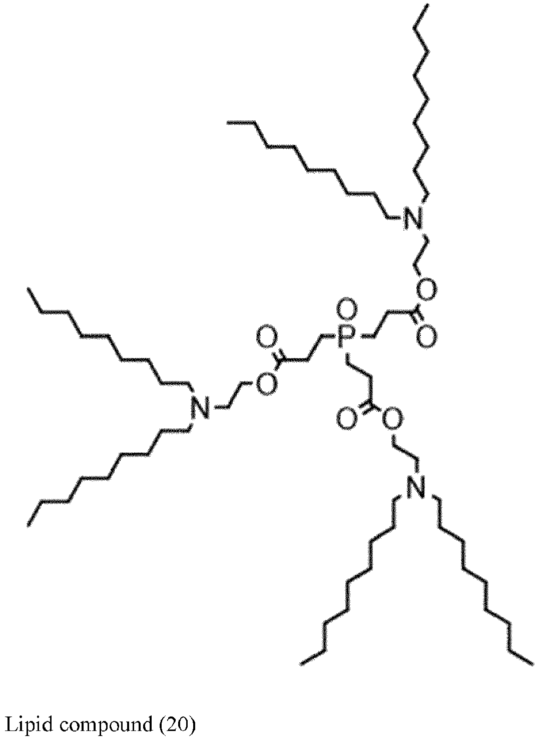 |
| Example 21 | 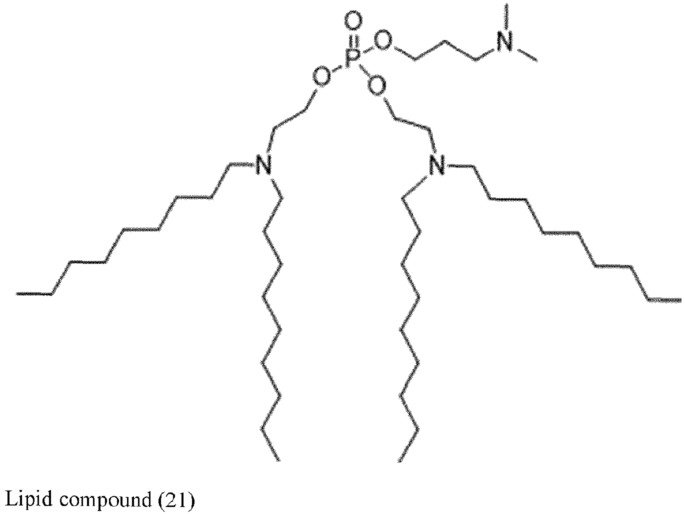 |
| Example 22 | 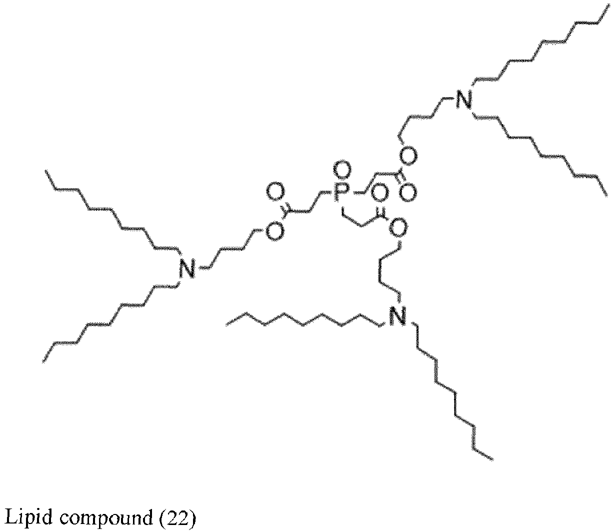 |
| Example 23 | 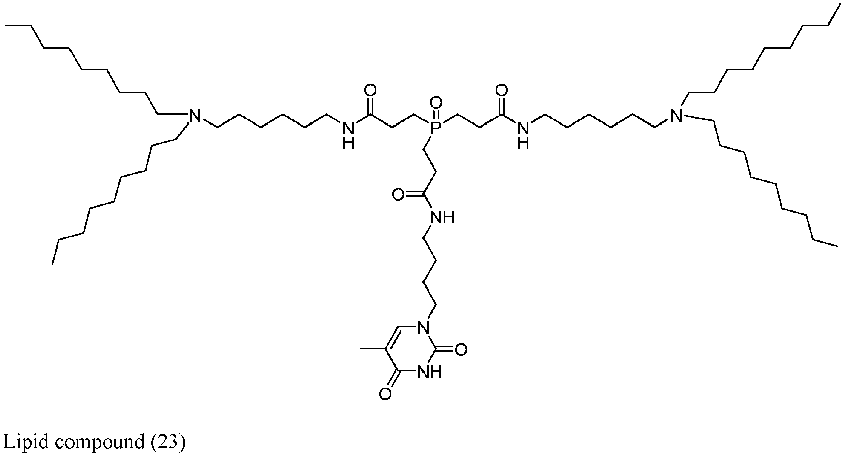 |
| Example 24 | 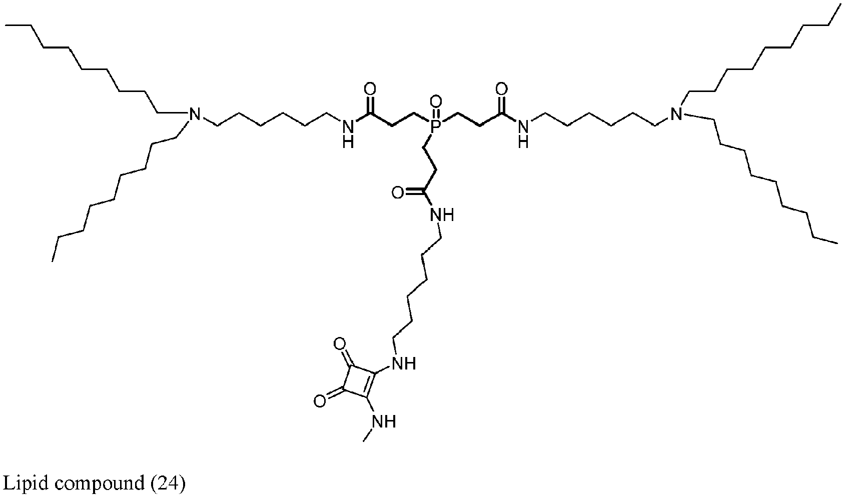 |
| Example 25 | 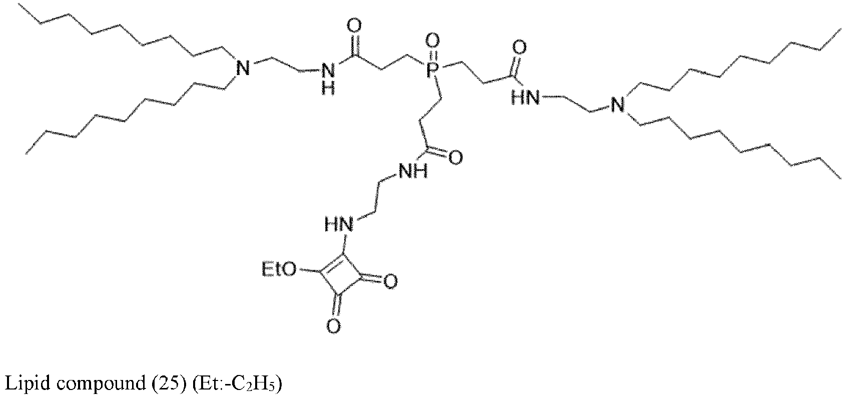 |
| Example 26 | 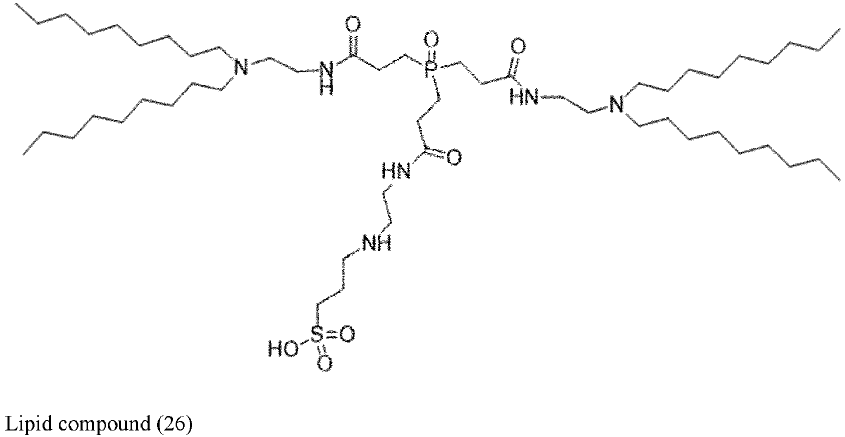 |
| Example 27 | 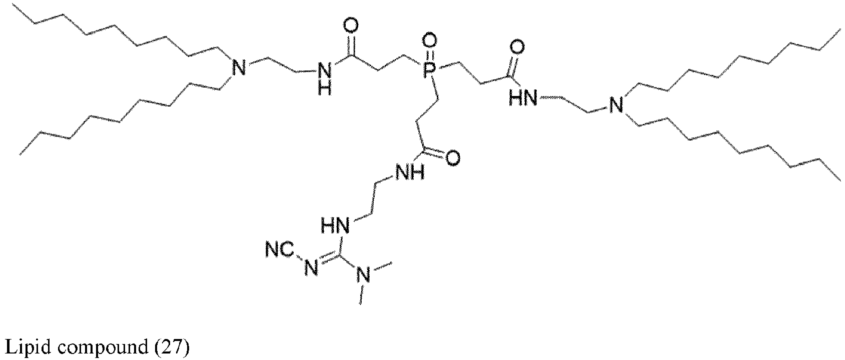 |
| Example 28 | 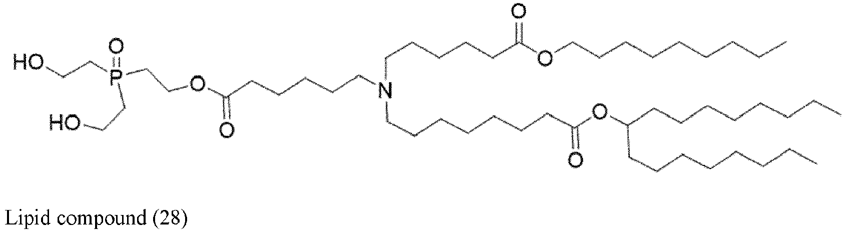 |
| Example 29 | 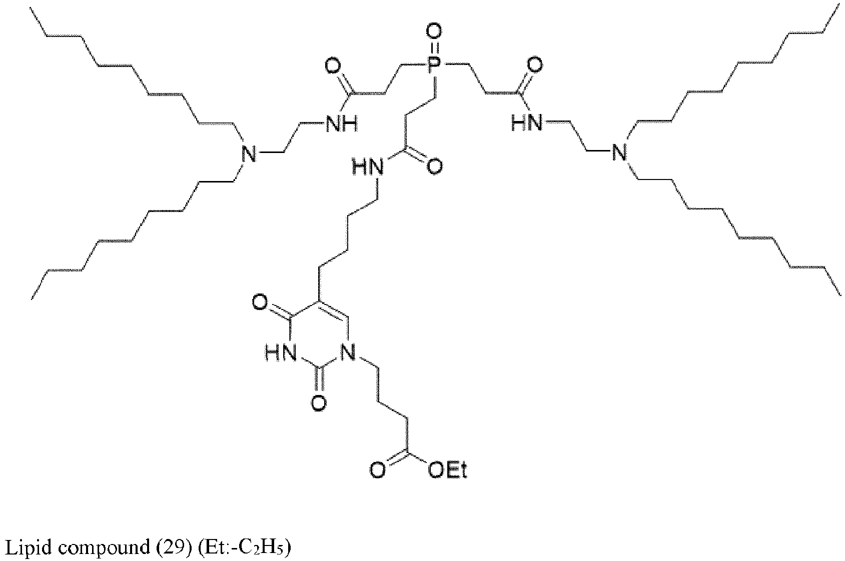 |
| Example 30 | 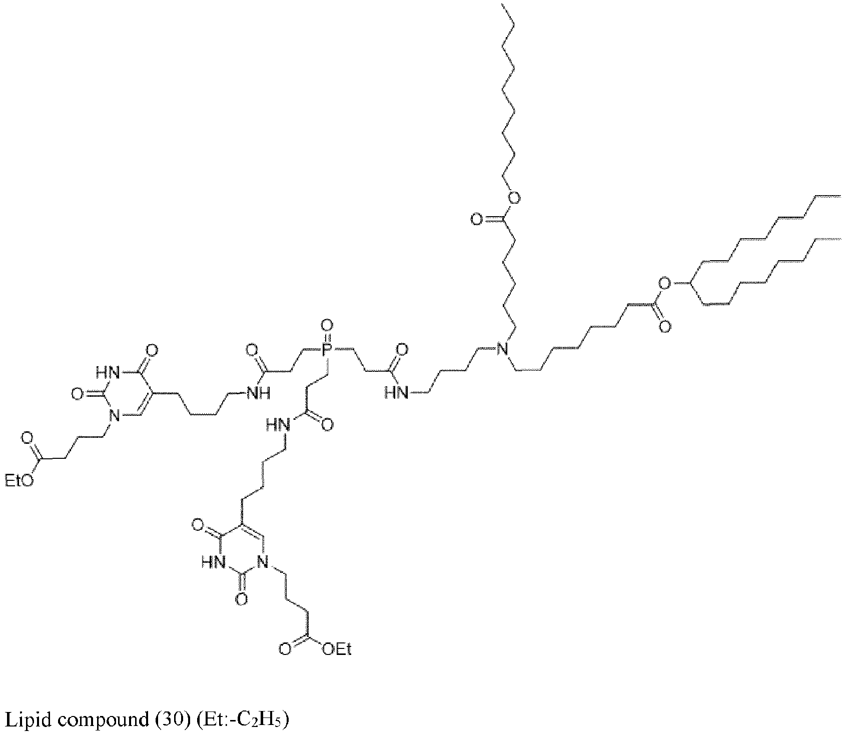 |
| Example 31 | 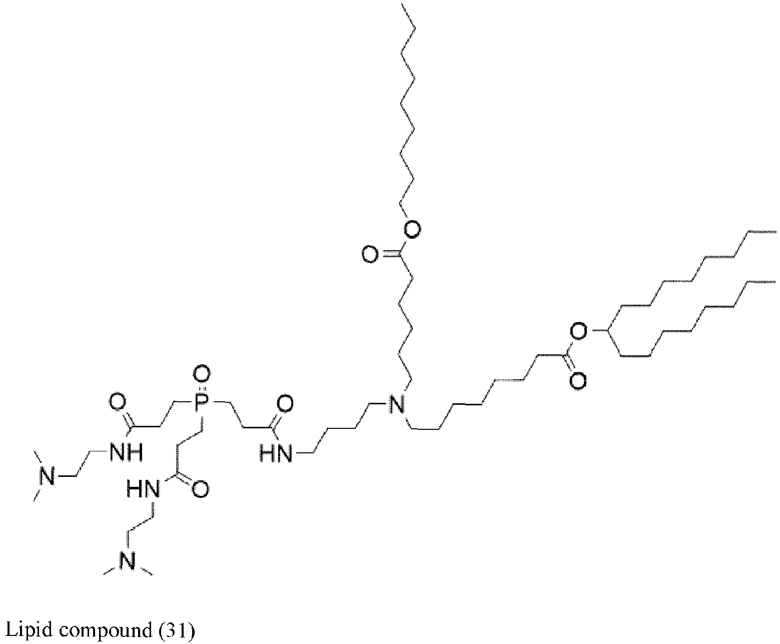 |
| Example 32 | 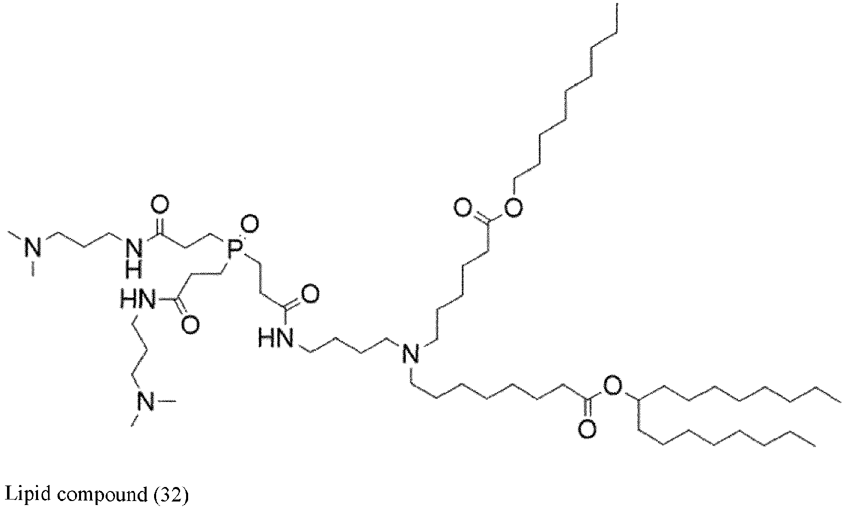 |
| Example 33 | 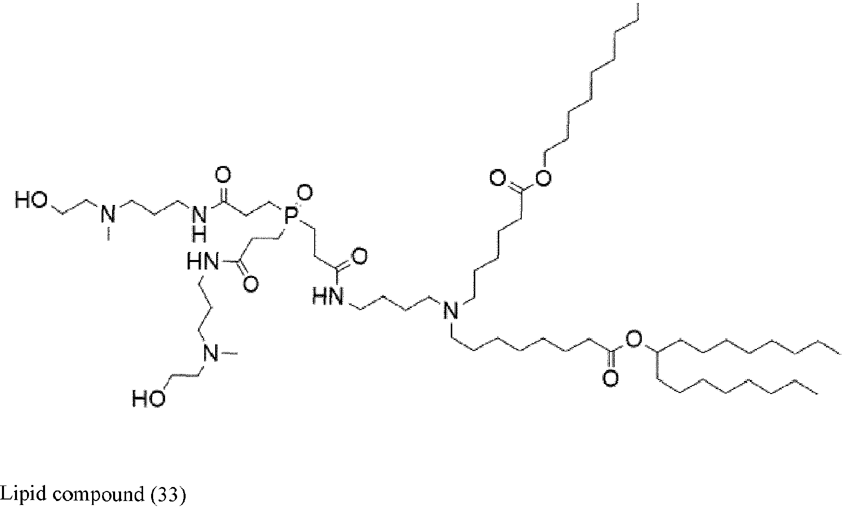 |
| Example 34 | 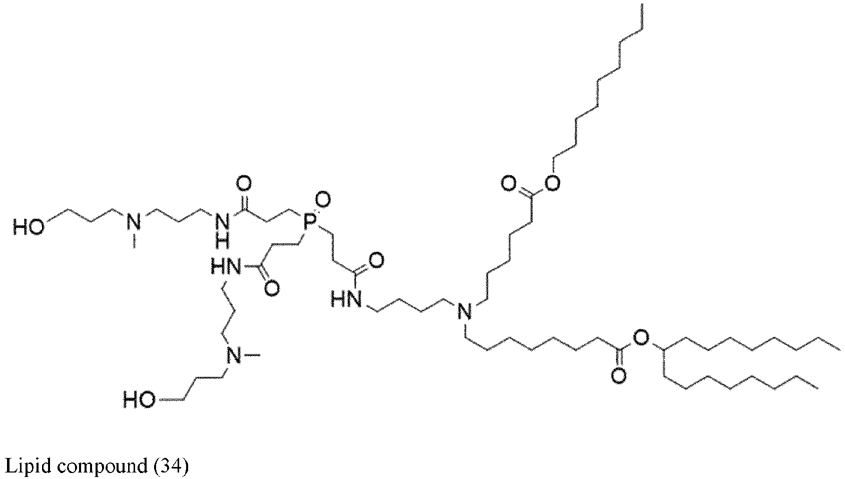 |

In order to further illustrate the preparation methods of the lipid compounds mentioned in the present disclosure, the preparation processes of the lipid compounds mentioned in Examples 1-3, 6-15, 18 and 19 are shown below to illustrate.

### Example 1

Compound (1) (2 equivalents) and tetrahydrofuran (THF) were added into a reaction flask under nitrogen to obtain a solution (concentration: 0.1 M). Next, a lithium bis(trimethylsilyl)amide (LiHMDS) solution (dissolved in tetrahydrofuran; the concentration was 1.0 M; LiHMDS was 3 equivalents) was added to the reaction flask. After stirring at room temperature for reacting for 1 hour, Compound (2) (1 equivalent) was added dropwise to the reaction flask. After the reaction was completed, the resultant was concentrated and extracted with n-hexane and brine. After collecting the organic phase, removing water with sodium sulfate (Na₂SO₄) and removing the solvent, the resultant was purified with high-performance liquid chromatography (HPLC) (using acetonitrile and trifluoroacetic acid (TFA) aqueous solution (the concentration was 0.1%) as eluent) to obtain Lipid compound (1). The reaction formula of the reaction mentioned above is as follows:

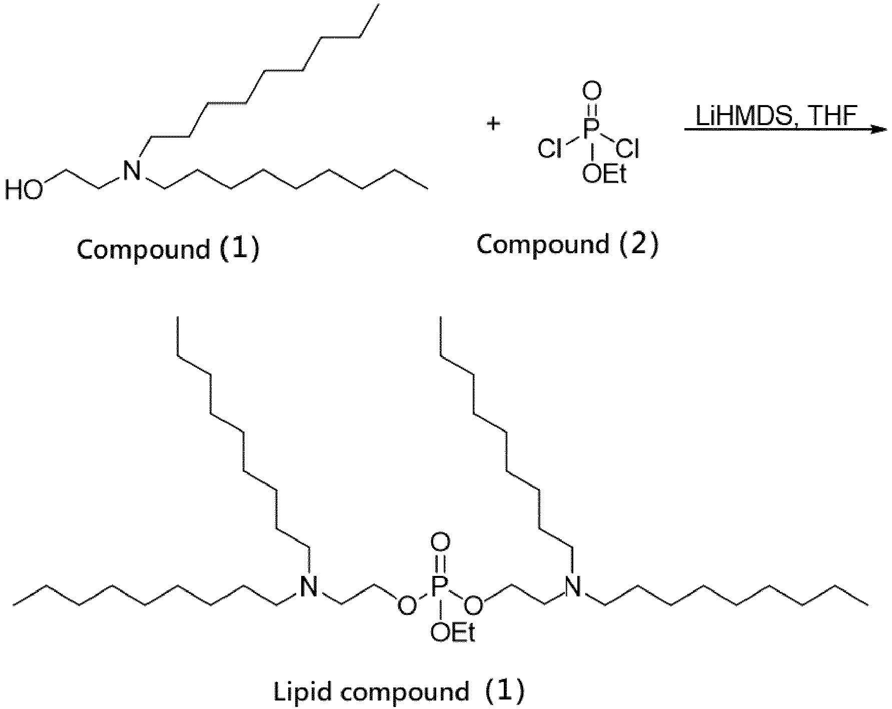

Next, Lipid compound (1) mentioned in Example 1 was analyzed by nuclear magnetic resonance spectroscopy. The obtained spectral information is shown in the following: ¹H-NMR (500 MHz, CDCl₃): δ 4.48 (s, 4H), 4.18 (t, *J* = 7.0, 7.5 Hz, 2H), 3.40 (s, 4H), 3.09 (m, 9H), 1.68 (s, 9H), 1.36-1.26 (m, 55H), 0.87 (t, *J* = 7.0, 7.0 Hz, 12H). After that, Lipid compound (1) was analyzed by liquid chromatography - mass spectrometry (LC-MS) and M/Z was measured: [M + H]⁺ = 718.7.

### Example 2

Compound (1) (2.5 equivalents) and tetrahydrofuran (THF) were added into a reaction flask under nitrogen to obtain a solution (concentration: 0.1 M). Next, a lithium bis(trimethylsilyl)amide (LiHMDS) solution (dissolved in tetrahydrofuran; the concentration was 1.0 M; LiHMDS was 3 equivalents) was added to the reaction flask. After stirring at room temperature for reacting for 1 hour, Compound (3) (1 equivalent) was added dropwise to the reaction flask. After the reaction was completed, the resultant was concentrated and extracted with n-hexane and brine. After collecting the organic phase, removing water with sodium sulfate (Na₂SO₄) and removing the solvent, the resultant was purified with high-performance liquid chromatography (HPLC) (using acetonitrile and trifluoroacetic acid (TFA) aqueous solution (the concentration was 0.1%) as eluent) to obtain Lipid compound (2). The reaction formula of the reaction mentioned above is as follows:

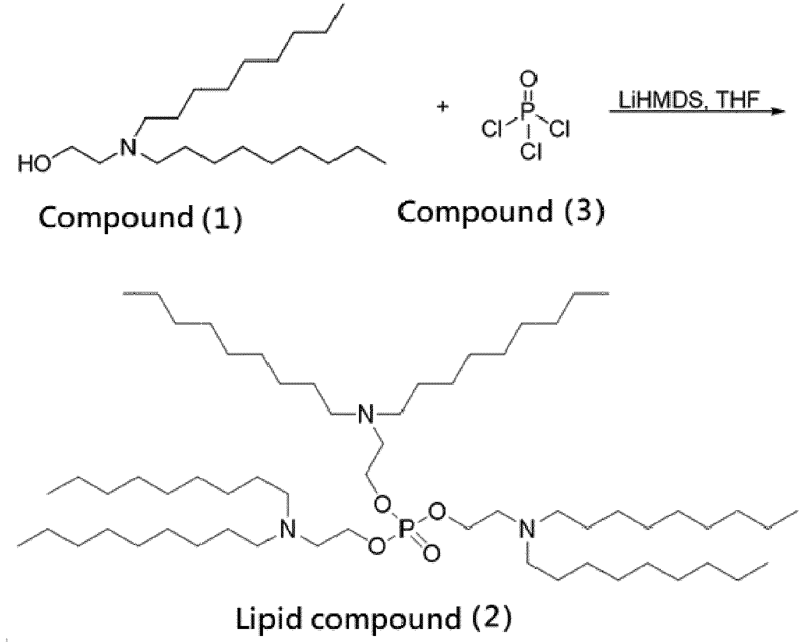

Next, Lipid compound (2) mentioned in Example 2 was analyzed by nuclear magnetic resonance spectroscopy. The obtained spectral information is shown in the following: ¹H-NMR (500 MHz, CDCl₃): δ 4.42 (s, 2H), 4.29 (s, 2H), 4.10-4.09 (m, 2H), 3.73 (s, 4H), 3.47-3.43 (m, 2H), 3.32-3.30 (m, 4H), 3.11-3.04 (m, 8H), 1.67 (s, 12H), 1.32-1.25 (m, 77H), 0.88 (t, *J* = 7.5, 7.5 Hz, 18H). After that, Lipid compound (2) was analyzed by liquid chromatography - mass spectrometry (LC-MS) and M/Z was measured: [M + H]⁺ = 986.1.

### Example 3

Compound (4) (2 equivalents) and tetrahydrofuran (THF) were added into a reaction flask under nitrogen to obtain a solution (concentration: 0.1 M). Next, a lithium bis(trimethylsilyl)amide (LiHMDS) solution (dissolved in tetrahydrofuran; the concentration was 1.0 M; LiHMDS was 3 equivalents) was added to the reaction flask. After stirring at room temperature for reacting for 1 hour, Compound (2) (1 equivalent) was added dropwise to the reaction flask. After the reaction was completed, the resultant was concentrated and extracted with n-hexane and brine. After collecting the organic phase, removing water with sodium sulfate (Na₂SO₄) and removing the solvent, the resultant was purified with high-performance liquid chromatography (HPLC) (using acetonitrile and trifluoroacetic acid (TFA) aqueous solution (the concentration was 0.1%) as eluent) to obtain Lipid compound (3). The reaction formula of the reaction mentioned above is as follows:

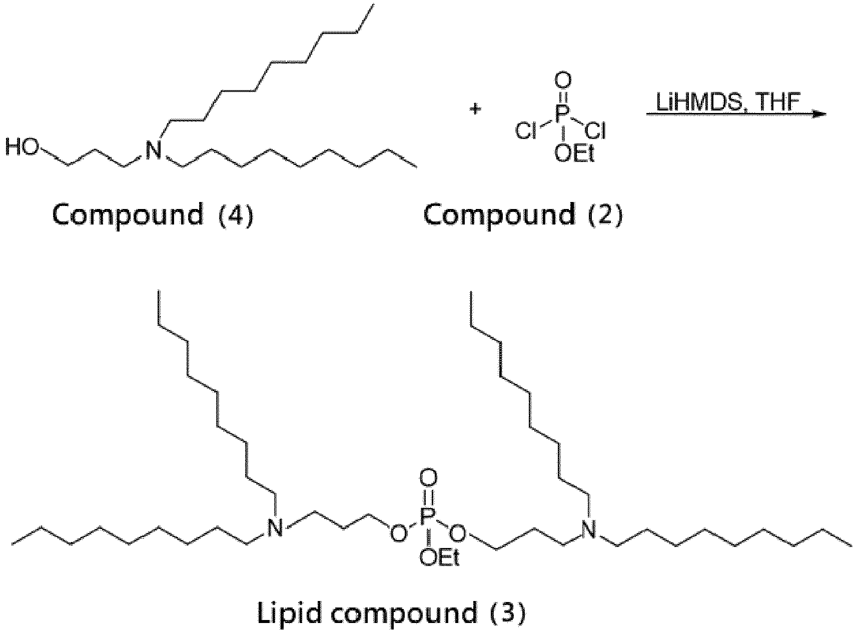

Next, Lipid compound (3) mentioned in Example 3 was analyzed by nuclear magnetic resonance spectroscopy. The obtained spectral information is shown in the following: ¹H-NMR (500 MHz, CDCl₃): 4.13 (t, *J* = 6.5, 7.5 Hz, 6H), 3.19 (s, 4H), 3.03-3.02 (m, 8H), 2.17-2.14 (m, 11H), 1.67 (s, 8H), 1.35-1.26 (m, 52H), 0.87 (t, *J* = 7.0, 6.5 Hz, 12H). After that, Lipid compound (3) was analyzed by liquid chromatography - mass spectrometry (LC-MS) and M/Z was measured: [M + H]⁺ = 746.5.

### Example 6

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2,3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (6) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (6). The reaction formula of the reaction mentioned above is as follows:

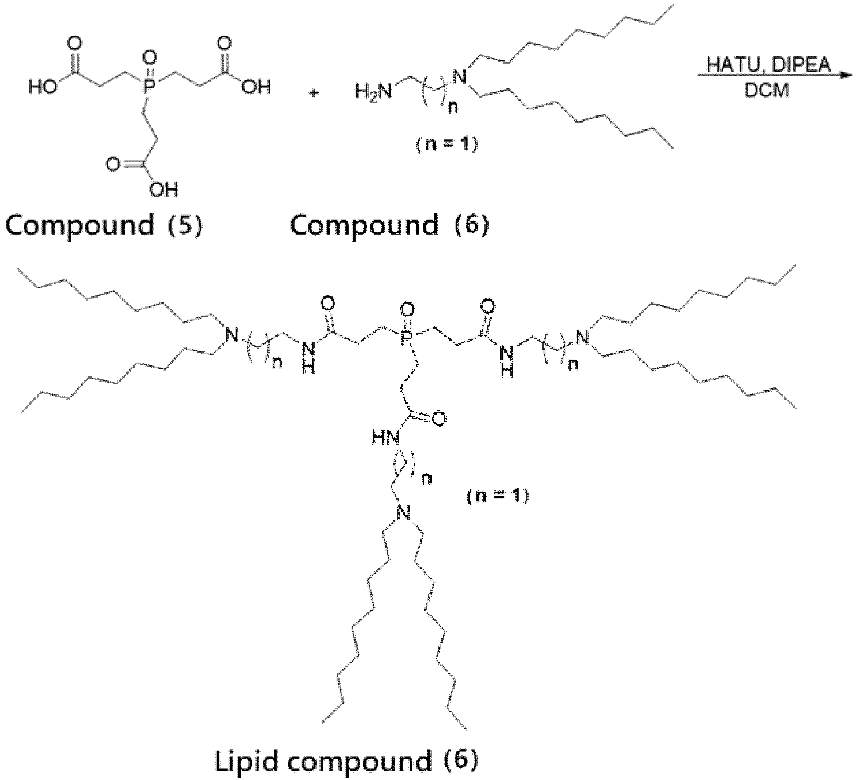

Next, Lipid compound (6) mentioned in Example 6 was analyzed by nuclear magnetic resonance spectroscopy. The obtained spectral information is shown in the following: ¹H-NMR (500 MHz, CDCl₃) : δ 10.62(s, 1H), 8.73 (s, 1H), 3.60 (s, 3H), 3.22 (s, 3H), 3.09-3.06 (m, 4H), 2.54-2.56 (m, 2H), 2.11-2.13 (m, 4H), 1.63 (s, 12H), 1.32-1.26 (m, 77H), 0.88 (t, J = 7.5, 7.5 Hz, 18H). After that, Lipid compound (6) was analyzed by liquid chromatography - mass spectrometry (LC-MS) and M/Z was measured: [M + H]⁺ = 1151.3.

### Example 7

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (7) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (7). The reaction formula of the reaction mentioned above is as follows:

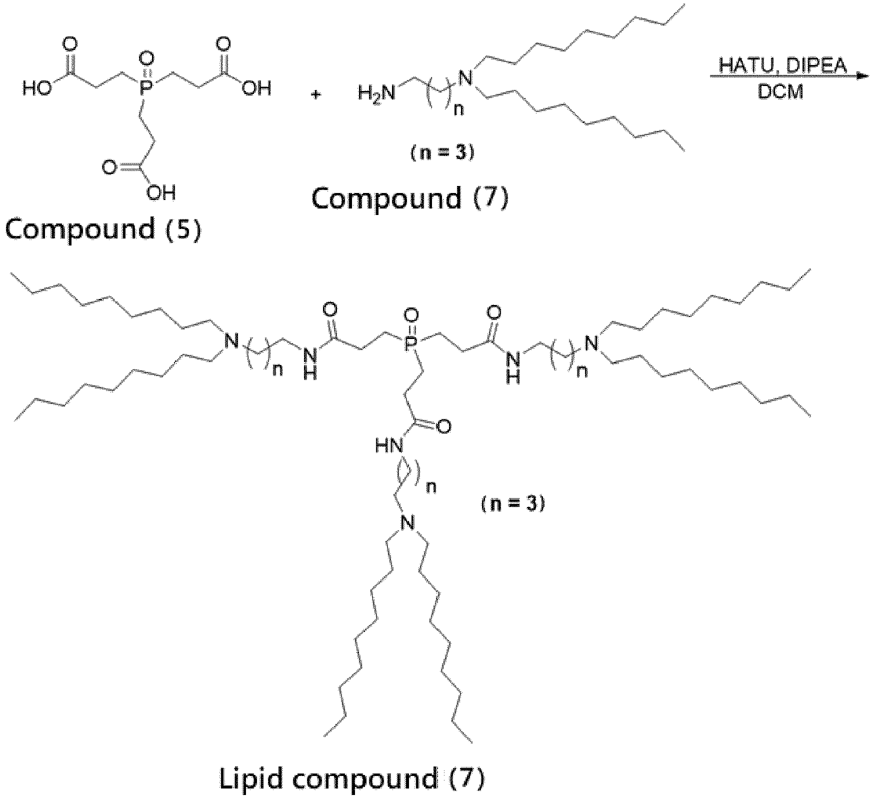

Next, Lipid compound (7) mentioned in Example 7 was analyzed by nuclear magnetic resonance spectroscopy. The obtained spectral information is shown in the following: ¹H-NMR (500 MHz, CDCl₃) : δ 7.98(s, 3H), 4.49 (s, 7H), 3.21 (s, 6H), 3.10-2.8 (m, 18H), 2.48 (s, 5H), 2.07 (s, 5H), 1.80-1.40 (m, 26H), 1.40-1.10 (m, 78H), 0.84 (t, *J* = 6.0, 7.0 Hz, 18H). After that, Lipid compound (7) was analyzed by liquid chromatography - mass spectrometry (LC-MS) and M/Z was measured: [M + H]⁺ = 1235.5.

### Example 8

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (8) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (8). The reaction formula of the reaction mentioned above is as follows:

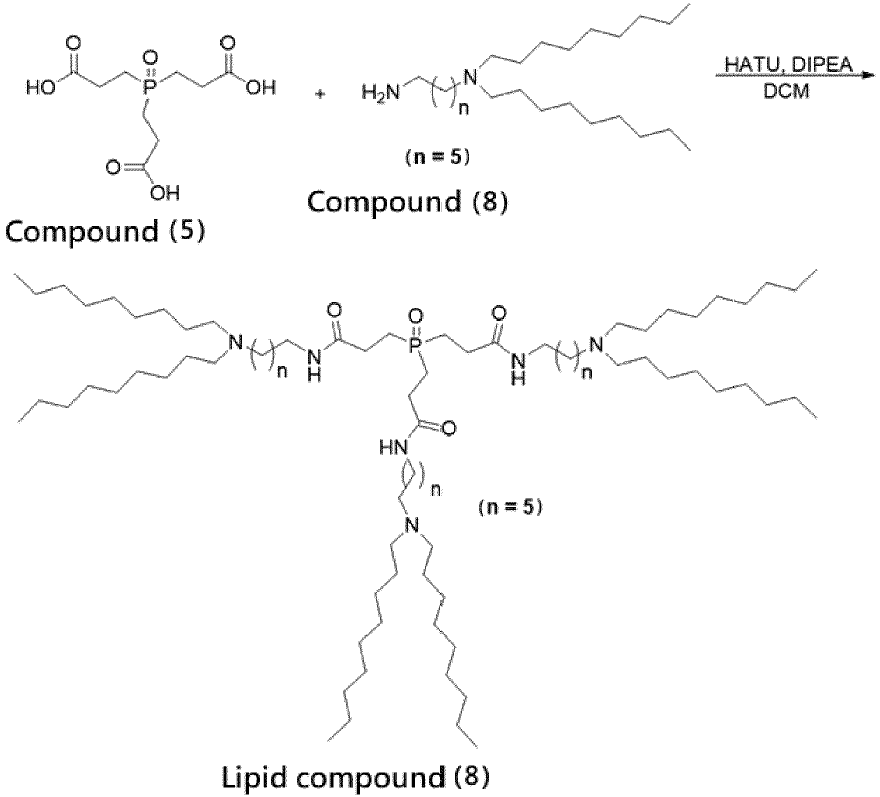

Next, Lipid compound (8) mentioned in Example 8 was analyzed by nuclear magnetic resonance spectroscopy. The obtained spectral information is shown in the following: ¹H-NMR (500 MHz, CDCl₃) : δ 11.52 (s, 1H), 5.57 (s, 1H), 3.18 (s, 7H), 2.30 (s, 10H), 2.50 (s, 3H), 2.06 (s, 4H), 1.67-1.65 (m, 11H), 1.34-1.25 (m, 51H), 0.87 (t, *J* = 2.0, 5.5 Hz, 9H). After that, Lipid compound (8) was analyzed by liquid chromatography - mass spectrometry (LC-MS) and M/Z was measured: [M + H]⁺ = 1319.6.

### Example 9

Compound (5) (1 equivalent) and dichloromethane were added into a reaction flask under nitrogen to obtain a solution (concentration: 0.05 M). Next, diisopropylethylamine (DIPEA) (3 equivalents) and N-[(dimethylamino)-1H-1, 2, 3-triazolo-[4, 5-b]pyridine-1-yl-methylene]-N-methylmethanaminium hexafluorophosphate N-oxide (HATU) (3 equivalents) were added to the reaction flask. After stirring at room temperature for reacting for 10 minutes, Compound (7) (4 equivalents) and Compound (9) (1 equivalent) were added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (9). The reaction formula of the reaction mentioned above is as follows:

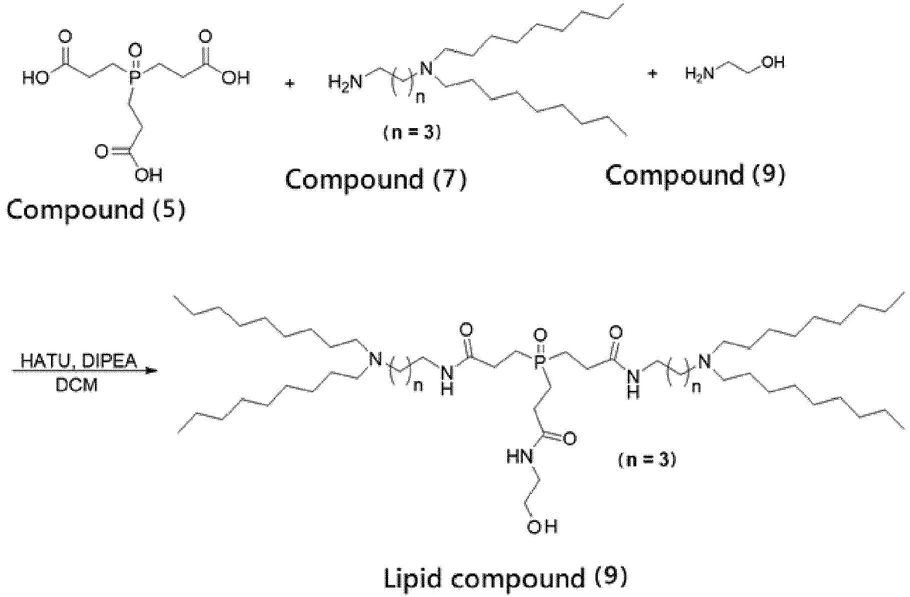

Next, Lipid compound (9) mentioned in Example 9 was analyzed by nuclear magnetic resonance spectroscopy. The obtained spectral information is shown in the following: ¹H-NMR (500 MHz, CDCl₃): ¹H-NMR (500 MHz, CDCl₃): δ 10.84 (s, 1H), 8.03 (s, 2H), 3.66 (s, 2H), 3.33-3.24 (m, 6H), 3.04-2.99 (m, 16H), 2.81 (s, 1H), 2.53 (s, 4H), 2.11 (s, 4H), 1.76-1.56 (m, 12H), 1.33-1.27 (m, 38H), 0.88 (t, *J* = 6.5, 7.5 Hz, 9H). After that, Lipid compound (9) was analyzed by liquid chromatography - mass spectrometry (LC-MS) and M/Z was measured: [M + H]⁺ = 955.8.

### Example 10

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1, 2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (10) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (10). The reaction formula of the reaction mentioned above is as follows:

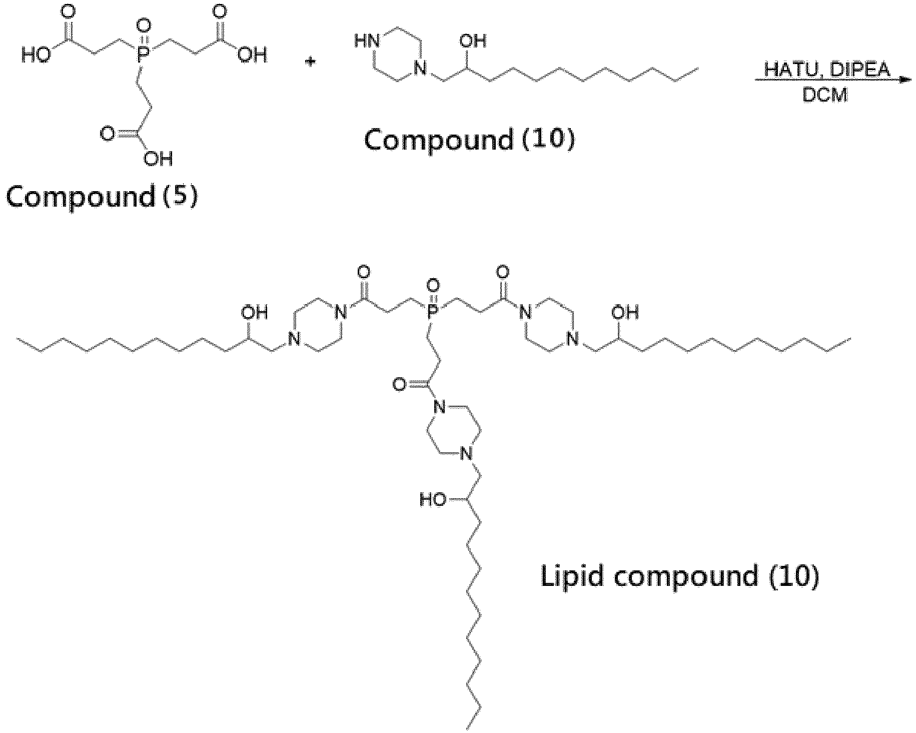

Next, Lipid compound (10) mentioned in Example 10 was analyzed by nuclear magnetic resonance spectroscopy. The obtained spectral information is shown in the following: ¹H-NMR (500 MHz, CDCl₃): δ 8.79 (s, 1H), 3.70-3.68 (m, 3H), 3.54 (s, 2H), 3.38 (s, 2H), 3.23-3.03 (m, 9H), 2.60-2.57 (m, 16H), 2.16-2.02 (m, 5H), 1.63 (s, 6H), 1.50 (t, *J* = 7.0, 7.5 Hz, 1H), 1.33-1.27 (m, 39H), 0.89 (t, *J* = 6.5, 7.0 Hz, 9H). After that, Lipid compound (10) was analyzed by liquid chromatography - mass spectrometry (LC-MS) and M/Z was measured: [M + H]⁺ = 1024.8.

### Example 11

Compound (5) (1 equivalent) and dichloromethane were added into a reaction flask under nitrogen to obtain a solution (concentration: 0.05 M). Next, diisopropylethylamine (DIPEA) (3 equivalents) and N-[(dimethylamino)-1H-1,2,3-triazolo-[4, 5-b]pyridine-1-yl-methylene]-N-methylmethanaminium hexafluorophosphate N-oxide (HATU) (3 equivalents) were added to the reaction flask. After stirring at room temperature for reacting for 10 minutes, Compound (6) (4 equivalents) and Compound (9) (1 equivalent) were added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (11). The reaction formula of the reaction mentioned above is as follows:

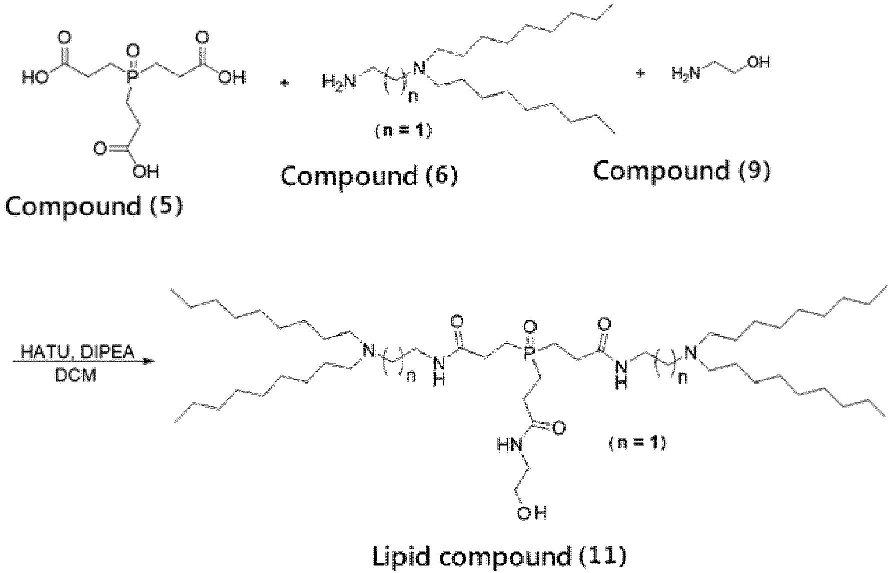

Next, Lipid compound (11) mentioned in Example 11 was analyzed by nuclear magnetic resonance spectroscopy. The obtained spectral information is shown in the following: ¹H-NMR (500 MHz, CDCl₃): δ 8.79 (s, 1H), 3.70-3.68 (m, 3H), 3.54 (s, 2H), 3.38 (s, 2H), 3.23-3.03 (m, 9H), 2.60-2.57 (m, 16H), 2.16-2.02 (m, 5H), 1.63 (s, 6H), 1.50 (t, *J* = 7.0, 7.5 Hz, 1H), 1.33-1.27 (m, 39H), 0.89 (t, *J* = 6.5, 7.0 Hz, 9H). After that, Lipid compound (11) was analyzed by liquid chromatography - mass spectrometry (LC-MS) and M/Z was measured: [M + H]⁺ = 899.7.

### Example 12

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (11) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (12). The reaction formula of the reaction mentioned above is as follows:

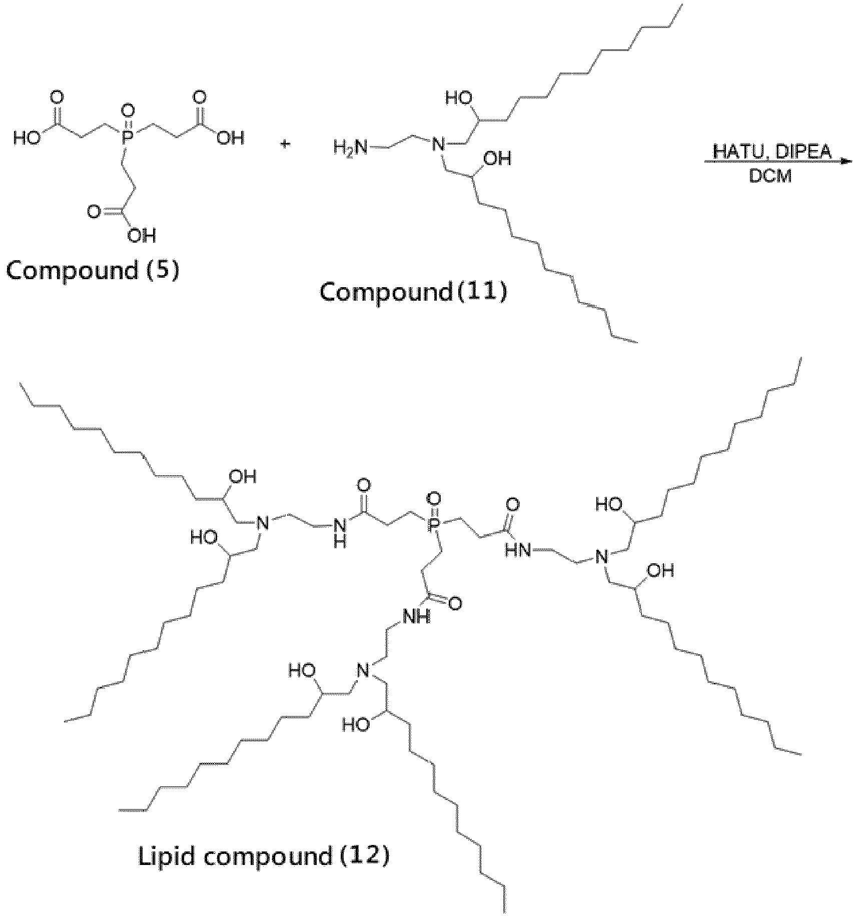

Next, Lipid compound (12) mentioned in Example 12 was analyzed by nuclear magnetic resonance spectroscopy. The obtained spectral information is shown in the following: ¹H-NMR (500 MHz, CDCl₃) : δ 8.50 (s, 2H), 6.46 (s, 5H), 4.10-3.90 (m, 3H), 3.90-3.0 (m, 13H), 2.55 (s, 3H), 2.15 (s, 3H), 1.60-1.20 (m, 61H), 0.88 (t, *J* = 7.0, 7.5 Hz, 10H). After that, Lipid compound (12) was analyzed by liquid chromatography - mass spectrometry (LC-MS) and M/Z was measured: [M + H]⁺ = 1499.4.

### Example 13

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (12) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (13). The reaction formula of the reaction mentioned above is as follows:

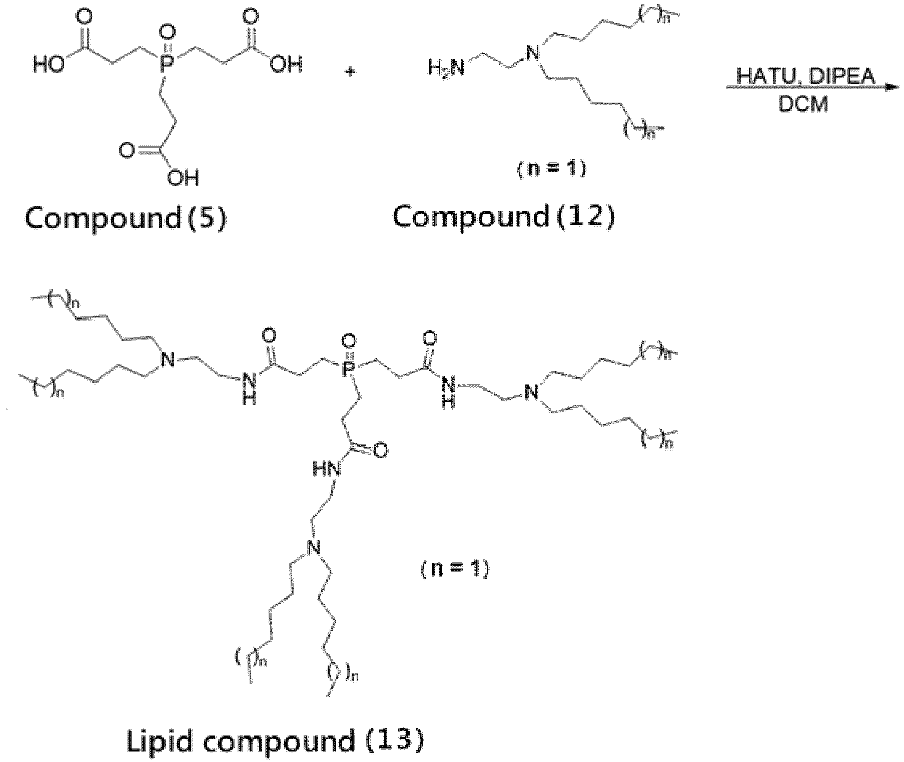

Next, Lipid compound (13) mentioned in Example 13 was analyzed by nuclear magnetic resonance spectroscopy. The obtained spectral information is shown in the following: ¹H-NMR (500 MHz, CDCl₃) : δ 10.68 (s, 2H), 8.78 (s, 3H), 3.59 (d, *J* = 4.0 Hz, 5H), 3.45-3.20 (m, 12H), 3.20-3.0 (m, 11H), 2.60-2.40 (m, 5H), 2.20-2.0 (m, 5H), 1.80-1.45 (m, 10H), 1.40-1.20 (m, 32H), 0.88 (t, *J* = 6.5 Hz, 15H). After that, Lipid compound (13) was analyzed by liquid chromatography - mass spectrometry (LC-MS) and M/Z was measured: [M + H]⁺ = 898.8.

### Example 14

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (13) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (14). The reaction formula of the reaction mentioned above is as follows:

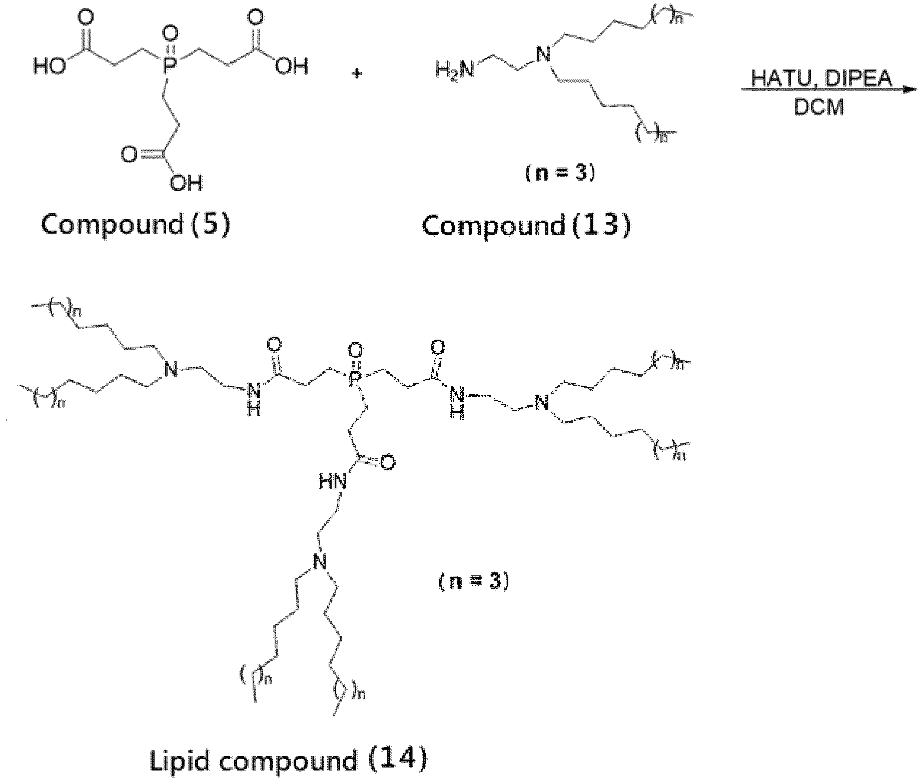

Next, Lipid compound (14) mentioned in Example 14 was analyzed by nuclear magnetic resonance spectroscopy. The obtained spectral information is shown in the following: ¹H-NMR (500 MHz, CDCl₃) : δ 10.67 (s, 2H), 8.75 (s, 2H), 4.25-4.0 (m, 5H), 3.62 (d, *J* = 4.5 Hz, 4H), 3.25-3.20 (m, 4H), 3.20-3.0 (m, 9H), 2.65-2.45 (m, 4H), 2.25-2.15 (m, 4H), 1.80-1.60 (m, 9H), 1.45-1.20 (m, 44H), 0.89 (t, *J* = 6.0, 7.5 Hz, 12H). After that, Lipid compound (14) was analyzed by liquid chromatography - mass spectrometry (LC-MS) and M/Z was measured: [M + H]⁺ = 1067.

### Example 15

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1, 2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (14) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (15). The reaction formula of the reaction mentioned above is as follows:

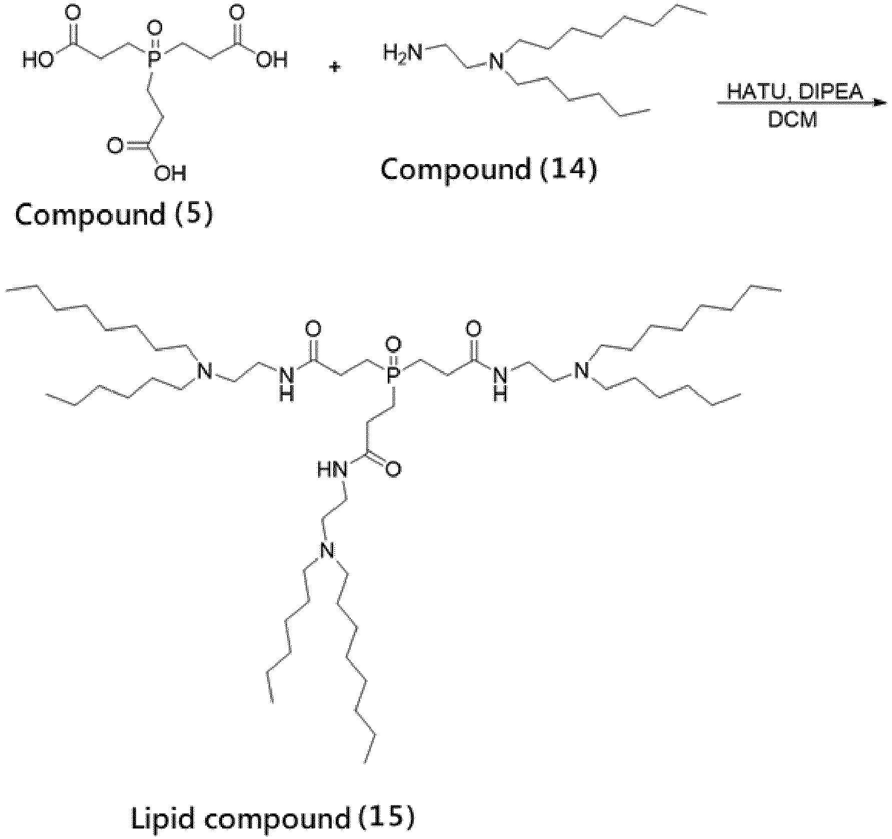

Next, Lipid compound (15) mentioned in Example 15 was analyzed by nuclear magnetic resonance spectroscopy. The obtained spectral information is shown in the following: ¹H-NMR (500 MHz, CDCl₃) : δ 10.77 (s, 2H), 8.80 (s, 2H), 3.62-3.50 (m, 7H), 3.30-3.20 (m, 5H), 3.20-3.0 (m, 11H), 2.60-2.50 (m, 5H), 2.20-2.0 (m, 5H), 1.80-1.50 (m, 11H), 1.50-1.20 (m, 40H), 0.89 (t, *J* = 5.0, 7.0 Hz, 14H). After that, Lipid compound (15) was analyzed by liquid chromatography - mass spectrometry (LC-MS) and M/Z was measured: [M + H]⁺ = 982.8.

### Example 18

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (15) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (18). The reaction formula of the reaction mentioned above is as follows:

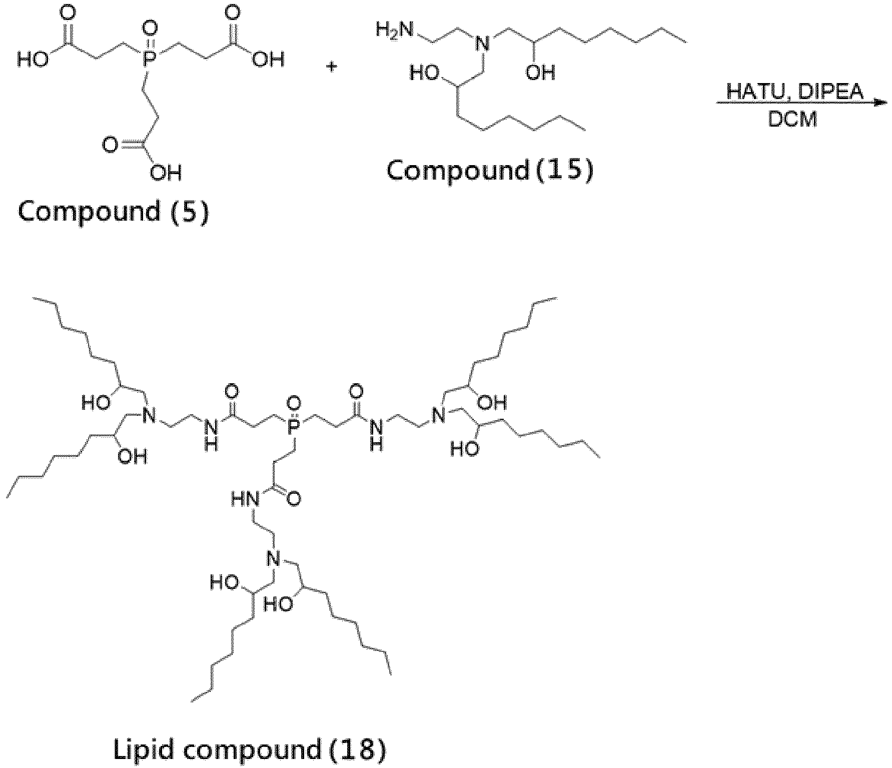

Next, Lipid compound (18) mentioned in Example 18 was analyzed by nuclear magnetic resonance spectroscopy. The obtained spectral information is shown in the following: ¹H-NMR (500 MHz, CDCl₃) : δ 8.66 (s, 2H), 4.60-3.90 (m, 13H), 3.90-3.0 (m, 9H), 2.70-2.40 (m, 4H), 2.30-2.0 (m, 4H), 1.70-1.20 (m, 45H), 0.89 (t, *J* = 6.0, 7.0 Hz, 13H). After that, Lipid compound (18) was analyzed by liquid chromatography - mass spectrometry (LC-MS) and M/Z was measured: [M + H]⁺ = 864.3.

### Example 19

Compound (5) (1 equivalent) and dichloromethane were added into a reaction flask under nitrogen to obtain a solution (concentration: 0.04 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (16) (4 equivalents) was added dropwise to the reaction flask. After reacting for 8 hours, the resultant was purified with high-performance liquid chromatography (HPLC) (using acetonitrile and trifluoroacetic acid (TFA) aqueous solution (the concentration was 0.1%) as eluent) to obtain Lipid compound (19). The reaction formula of the reaction mentioned above is as follows:

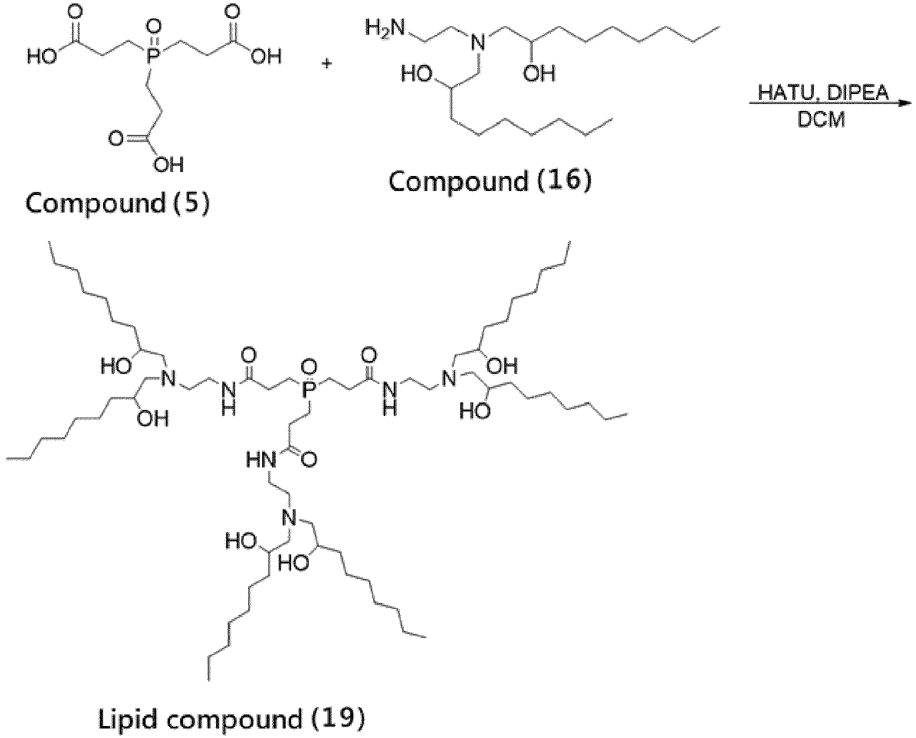

Next, Lipid compound (19) mentioned in Example 19 was analyzed by nuclear magnetic resonance spectroscopy. The obtained spectral information is shown in the following: ¹H-NMR (500 MHz, CDCl₃): δ 8.63 (s, 2H), 4.70-3.90 (m, 12H), 3.90-3.0 (m, 17H), 2.70-2.50 (m, 4H), 2.30-2.10 (m, 4H), 1.60-1.20 (m, 48H), 0.88 (t, *J* = 6.5, 7.5 Hz, 11H). After that, Lipid compound (19) was analyzed by liquid chromatography - mass spectrometry (LC-MS) and M/Z was measured: [M + H]⁺ = 1247.1.

### Example 23

Compound (23a) (1 equivalent) and dichloromethane were added into a reaction flask under nitrogen to obtain a solution (concentration: 0.05 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (17) (2.5 equivalents) was added dropwise to the reaction flask. After reacting for 12 hours, the resultant was purified with high-performance liquid chromatography (HPLC) (using acetonitrile and trifluoroacetic acid (TFA) aqueous solution (the concentration was 0.1%) as eluent) to obtain Lipid compound (23). The reaction formula of the reaction mentioned above is as follows:

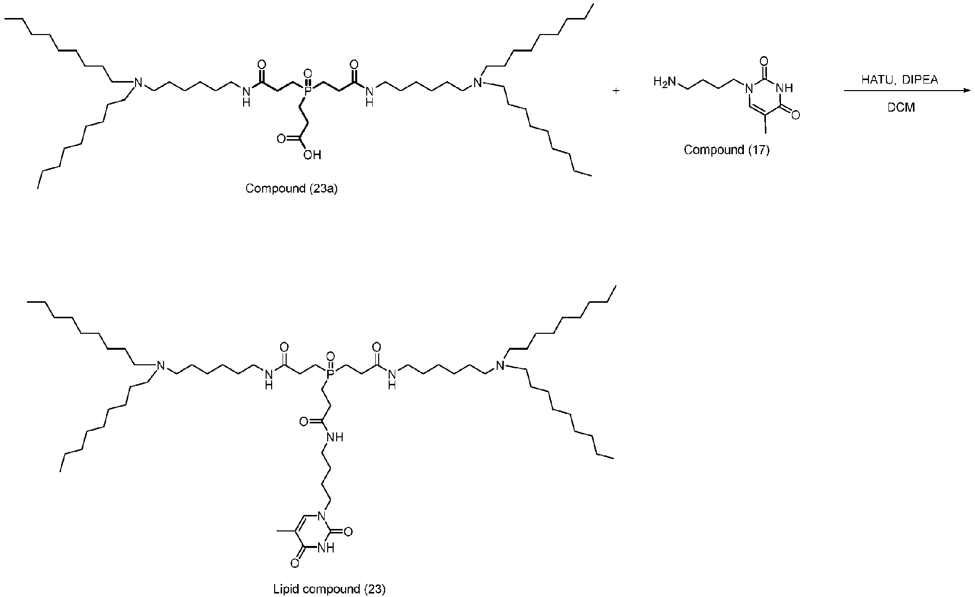

Next, Lipid compound (23) mentioned in Example 23 was analyzed by liquid chromatography - mass spectrometry (LC-MS) and M/Z was measured: [M + H]⁺ = 1147.6.

### Example 24

Compound (24a) (1 equivalent) and dichloromethane were added into a reaction flask under nitrogen to obtain a solution (concentration: 0.05 M). Next, diisopropylethylamine (DIPEA) (4 equivalents) and N-[(dimethylamino)-1H-1,2, 3-triazolo-[4, 5-b]pyridine-1-yl-methylene]-N-methylmethanaminium hexafluorophosphate N-oxide (HATU) (4 equivalents) were added to the reaction flask. After stirring at room temperature for reacting for 10 minutes, Compound (18) (2.5 equivalents) was added dropwise to the reaction flask. After reacting for 16 hours, the resultant was purified with high-performance liquid chromatography (HPLC) (using acetonitrile and trifluoroacetic acid (TFA) aqueous solution (the concentration was 0.1%) as eluent) to obtain Lipid compound (24). The reaction formula of the reaction mentioned above is as follows:

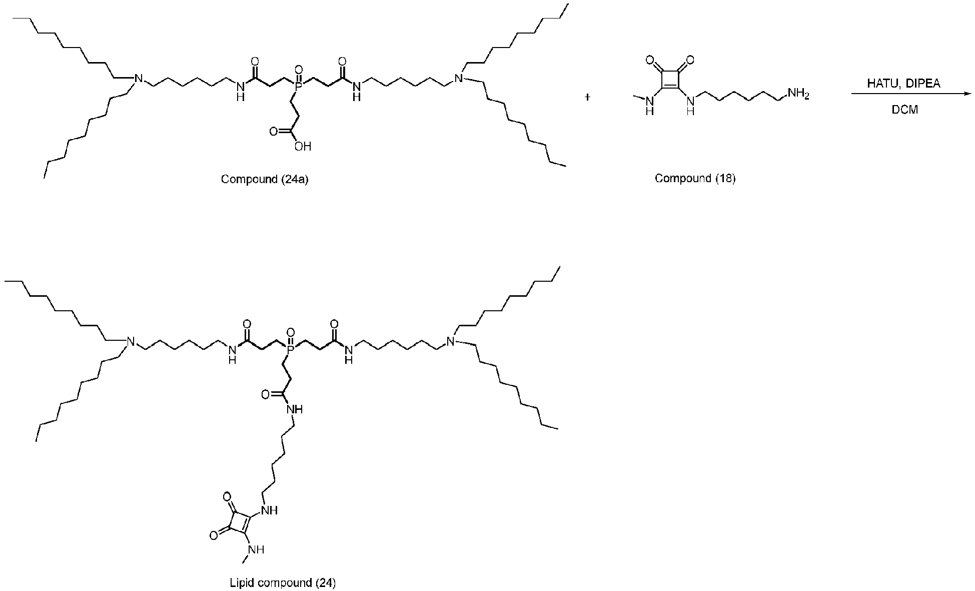

Next, Lipid compound (24) mentioned in Example 24 was analyzed by liquid chromatography - mass spectrometry (LC-MS) and M/Z was measured: [M + H]⁺ = 1175.7.

### Preparation of compositions

### Example 35

Luciferase mRNA (product number R1018, purchased from APExBIO) was dissolved in an acidic buffer (product number J63669, purchased from Alfa Aesar) (pH value 4.5) to obtain a nucleic acid aqueous solution (concentration: 0.1 mg/mL).

Lipid compound (1) prepared in Example 1, cholesterol, 1, 2-distearoyl-sn-glycero-3-phosphocholine (DSPC), and DMG-PEG lipid (1, 2-dimyristoyl-rac-glycero-3-methoxypolyethylene glycol)(product number: DMG-PEG-2K, purchased from Nippon Fine Chemical) were dissolved in ethanol to obtain a lipid solution (concentration: 10-15 mg/mL), wherein the contents of Lipid compound (1), cholesterol, DSPC and DMG-PEG lipid contents are shown in Table 2.

Next, the nucleic acid aqueous solution and the lipid solution were mixed through a microfluidic system (NanoAssemblr Ignite system, purchased from Precision NanoSystem Inc.), and the volumes of the nucleic acid aqueous solution and the lipid solution were adjusted so that the ratio of nitrogen atoms (N) of Lipid compound (1) to phosphorus atoms (P) derived from luciferase mRNA (N/P) was 12. Then, after ultrafiltration or dialysis, the obtained composition was self-assembled to form Nucleic acid-lipid nanoparticle carrier (1).

### Examples 36-43

Nucleic acid-lipid nanoparticle carriers (2)-(9) were obtained by performing the same manner as in Example 35 but changing the ingredients and/or contents in the lipid solution and the N/P value (adjusted by the volume ratio of the nucleic acid aqueous solution and the lipid solution) according to Table 1.

### Comparative example 1

Nucleic acid-lipid nanoparticle carrier (10) was obtained by performing the same manner as in Example 35 but replacing Lipid compound (1) with (6Z, 9Z, 28Z, 31Z)-heptatriacont-6, 9, 28, 31-tetraene-19-yl 4-(dimethylamino)butanoate (DLin-MC3-DMA) and adjusting the N/P value (adjusted by the volume ratio of the nucleic acid aqueous solution and the lipid solution).

### Comparative example 2

Nucleic acid-lipid nanoparticle carrier (11) was obtained by performing the same manner as in Example 35 but replacing Lipid compound (1) with heptadecan-9-yl 8-((2-hydroxyethyl)(6-oxo-6-(undecyloxy)hexyl)amino)octanoate (SM-102) and adjusting the N/P value (adjusted by the volume ratio of the nucleic acid aqueous solution and the lipid solution).

**Table 2**

| | | N/P | Lipid compound (1) (parts by mole) | DMG-PEG (parts by mole) | DSPC (parts by mole) | Cholesterol (parts by mole) |
|---|---|---|---|---|---|---|
| Example 35 | Nucleic acid-lipid nanoparticle carrier (1) | 12 | 100 | 3 | 20 | 77 |
| Example 36 | Nucleic acid-lipid nanoparticle carrier (2) | 12 | 100 | 2.5 | 0 | 64.17 |
| Example 37 | Nucleic acid-lipid nanoparticle carrier (3) | 12 | 100 | 3.75 | 50 | 96.25 |
| Example 38 | Nucleic acid-lipid nanoparticle carrier (4) | 12 | 100 | 7.15 | 45.71 | 132.86 |

| | | N/P | Lipid compound (2) (parts by mole) | DMG-PEG (parts by mole) | DSPC (parts by mole) | Cholesterol (parts by mole) |
|---|---|---|---|---|---|---|
| Example 39 | Nucleic acid-lipid nanoparticle carrier (5) | 18 | 100 | 3.75 | 50 | 96.25 |
| Example 40 | Nucleic acid-lipid nanoparticle carrier (6) | 18 | 100 | 7.15 | 45.71 | 132.86 |
| Example 41 | Nucleic acid-lipid nanoparticle carri er (7) | 9 | 100 | 4.55 | 150 | 200 |

| | | N/P | Lipid compound (1)(parts by mole)/Lipid compound (2)(parts by mole) | DMG-PEG (parts by mole) | DSPC (parts by mole) | Cholesterol (parts by mole) |
|---|---|---|---|---|---|---|
| Example 42 | Nucleic acid-lipid nanoparticle carrier (8) | 16.5 | 75/25 | 3.75 | 50 | 96.25 |
| Example 43 | Nucleic acid-lipid nanoparticle carrier (9) | 17.3 | 87.5/12.5 | 3.75 | 50 | 96.25 |

| | | N/P | DLin-MC3-DMA | DMG-PEG (parts by mole) | DSPC (parts by mole) | Cholesterol (parts by mole) |
|---|---|---|---|---|---|---|
| Comparative example 1 | Nucleic acid-lipid nanoparticle carrier (10) | 6 | 100 | 3 | 20 | 77 |

| | | N/P | SM-102 | DMG-PEG (parts by mole) | DSPC (parts by mole) | Cholesterol (parts by mole) |
|---|---|---|---|---|---|---|
| Comparative example 2 | Nucleic acid-lipid nanoparticle carrier (11) | 6 | 100 | 3 | 20 | 77 |

### Examples 44-52

Nucleic acid-lipid nanoparticle carriers (12)-(20) were obtained by performing the same manner as in Example 35 but changing the ingredients and/or contents in the lipid solution and the N/P value (adjusted by the volume ratio of the nucleic acid aqueous solution and the lipid solution) according to Table 3.

**Table 3**

| | | N/P | Lipid compound (3) (parts by mole) | DMG-PEG (parts by mole) | DSPC (parts by mole) | Cholesterol (parts by mole) |
|---|---|---|---|---|---|---|
| Example 44 | Nucleic acid-lipid nanoparticle carrier (12) | 12 | 100 | 3.75 | 50 | 96.25 |
| Example 45 | Nucleic acid-lipid nanoparticle carrier (13) | 12 | 100 | 7.15 | 45.71 | 132.86 |
| Example 46 | Nucleic acid-lipid nanoparticle carrier (14) | 16 | 100 | 7.15 | 45.71 | 132.86 |

| | | N/P | Lipid compound (6) (parts by mole) | DMG-PEG (parts by mole) | DSPC (parts by mole) | Cholesterol (parts by mole) |
|---|---|---|---|---|---|---|
| Example 47 | Nucleic acid-lipid nanoparticle carrier (15) | 18 | 100 | 3 | 20 | 77 |
| Example 48 | Nucleic acid-lipid nanoparticle carrier (16) | 18 | 100 | 5 | 100 | 128.33 |
| Example 49 | Nucleic acid-lipid nanoparticle carrier (17) | 18 | 100 | 7.15 | 45.71 | 132.86 |

| | | N/P | Lipid compound (7) (parts by mole) | DMG-PEG (parts by mole) | DSPC (parts by mole) | Cholesterol (parts by mole) |
|---|---|---|---|---|---|---|
| Example 50 | Nucleic acid-lipid nanoparticle carrier (18) | 18 | 100 | 3 | 20 | 77 |
| Example 51 | Nucleic acid-lipid nanoparticle carrier (19) | 18 | 100 | 5 | 100 | 128.33 |
| Example 52 | Nucleic acid-lipid nanoparticle carrier (20) | 18 | 100 | 7.15 | 45.71 | 132.86 |

### Examples 53-61

Nucleic acid-lipid nanoparticle carriers (21)-(29) were obtained by performing the same manner as in Example 35 but changing the ingredients and/or contents in the lipid solution and the N/P value (adjusted by the volume ratio of the nucleic acid aqueous solution and the lipid solution) according to Table 4.

**Table 4**

| | | N/P | Lipid compound (8) (parts by mole) | DMG-PEG (parts by mole) | DSPC (parts by mole) | Cholesterol (parts by mole) |
|---|---|---|---|---|---|---|
| Example 53 | Nucleic acid-lipid nanoparticle carrier (21) | 18 | 100 | 3 | 20 | 77 |
| Example 54 | Nucleic acid-lipid nanoparticle carrier (22) | 18 | 100 | 5 | 100 | 128.33 |
| Example 55 | Nucleic acid-lipid nanoparticle carrier (23) | 18 | 100 | 7.15 | 45.71 | 132.86 |
| Example 56 | Nucleic acid-lipid nanoparticle carrier (24) | 6 | 100 | 3 | 20 | 77 |
| Example 57 | Nucleic acid-lipid nanoparticle carrier (25) | 6 | 100 | 5 | 100 | 128.33 |
| Example 58 | Nucleic acid-lipid nanoparticle carrier (26) | 6 | 100 | 7.15 | 45.71 | 132.86 |

| | | N/P | Lipid compound (9) (parts by mole) | DMG-PEG (parts by mole) | DSPC (parts by mole) | Cholesterol (parts by mole) |
|---|---|---|---|---|---|---|
| Example 59 | Nucleic acid-lipid nanoparticle carrier (27) | 12 | 100 | 3 | 20 | 77 |
| Example 60 | Nucleic acid-lipid nanoparticle carrier (28) | 12 | 100 | 5 | 100 | 128.33 |
| Example 61 | Nucleic acid-lipid nanoparticle carrier (29) | 12 | 100 | 7.15 | 45.71 | 132.86 |

### Examples 62-70

Nucleic acid-lipid nanoparticle carriers (30)-(38) were obtained by performing the same manner as in Example 35 but changing the ingredients and/or contents in the lipid solution and the N/P value (adjusted by the volume ratio of the nucleic acid aqueous solution and the lipid solution) according to Table 5.

**Table 5**

| | | N/P | Lipid compound (9) (parts by mole) | DMG-PEG (parts by mole) | DSPC (parts by mole) | Cholesterol (parts by mole) |
|---|---|---|---|---|---|---|
| Example 62 | Nucleic acid-lipid nanoparticle carrier (30) | 6 | 100 | 3 | 20 | 77 |
| Example 63 | Nucleic acid-lipid nanoparticle carrier (31) | 6 | 100 | 5 | 100 | 128.33 |
| Example 64 | Nucleic acid-lipid nanoparticle carrier (32) | 6 | 100 | 7.15 | 45.71 | 132.86 |

| | | N/P | Lipid compound (10) (parts by mole) | DMG-PEG (parts by mole) | DSPC (parts by mole) | Cholesterol (parts by mole) |
|---|---|---|---|---|---|---|
| Example 65 | Nucleic acid-lipid nanoparticle carrier (33) | 18 | 100 | 3 | 20 | 77 |
| Example 66 | Nucleic acid-lipid nanoparticle carrier (34) | 18 | 100 | 5 | 100 | 128.33 |
| Example 67 | Nucleic acid-lipid nanoparticle carrier (35) | 18 | 100 | 7.15 | 45.71 | 132.86 |

| | | N/P | Lipid compound (11) (parts by mole) | DMG-PEG (parts by mole) | DSPC (parts by mole) | Cholesterol (parts by mole) |
|---|---|---|---|---|---|---|
| Example 68 | Nucleic acid-lipid nanoparticle carrier (36) | 12 | 100 | 3 | 20 | 77 |
| Example 69 | Nucleic acid-lipid nanoparticle carrier (37) | 12 | 100 | 5 | 100 | 128.33 |
| Example 70 | Nucleic acid-lipid nanoparticle carrier (38) | 12 | 100 | 7.15 | 45.71 | 132.86 |

### Property evaluation of pharmaceutical compositions

Nucleic acid-lipid nanoparticle carriers (1)-(9) obtained in Examples 35-43 and Nucleic acid-lipid nanoparticle carriers (10) and (11) obtained in Comparative examples 1 and 2 were evaluated for particle size, polydispersity index (PdI), Zeta potential, ribonucleic acid recovery rate (mRNA recovery), encapsulation efficiency (EE) for ribonucleic acid and acid dissociation constant (pKa). The results are shown in Table 6.

The particle size, polydispersity index (PdI) and Zeta potential of nucleic acid-lipid nanoparticle carriers are evaluated as follows. 20 µL of the sample was taken to add to 330 µL of Dulbecco's Phosphate-Buffered Saline (DPBS) and shaken evenly, and then measured for particle size and polydispersity index (PdI) of liposomes with a dynamic light scattering instrument (Zetasizer Nano-ZS, Malvern Panalytical). In addition, 20 µL of the sample was taken to add to 680 µL of sodium chloride aqueous solution (concentration: 10 mM), shaken evenly, and then measured for Zeta potential of liposomes with a dynamic light scattering instrument (Zetasizer Nano-ZS, Malvern Panalytical).

The ribonucleic acid recovery rate (mRNA recovery) is evaluated as follows. After sterile filtration of the nucleic acid lipid nanoparticle carriers, 100 µL of sample was taken to add to 25 µL of 5% Triton X-100. Next, after incubating at 37°C for 10 minutes, 100 µL of chloroform was added thereto. After high-speed shaking (rotation speed: 2000 rpm) for 10 minutes and centrifugation (centrifugal force: 14000 ×g) for 30 minutes, 80 µL of the supernatant was taken and its absorbance was analyzed by a UV-visible spectrophotometer (UV7, Mettler Toledo). After that, the concentration of ribonucleic acid was calculated using Formula (1) listed below, and the recovery rate was calculated using Formula (2) listed below, wherein C is the concentration of ribonucleic acid, A₂₆₀ₙₘ is the absorbance value of ribonucleic acid at a wavelength of 260 nm, Vf is the volume of supernatant (µL), and W is the initial weight of ribonucleic acid (ng). $C = \left({A}_{260 nm}\times 40\right) / \left(100/125\right)$ $Recovery rate \left(%\right) = \left[\left(C\times Vf\right)/W\right] \times 100 %$

The encapsulation efficiency (EE) for ribonucleic acid was measured by the Quant-iT RiboGreen RNA quantitative detection kit (RNA Assay kit) (purchased from Invitrogen). Namely, the mRNA concentration in the dispersion containing nucleic acid-lipid particles was quantified in the presence and absence of 0.2% Triton X-100 surfactant, and the encapsulation efficiency (EE) for ribonucleic acid was calculated according to Formula (3) listed below, wherein EE is the encapsulation efficiency for ribonucleic acid, C_{Free} is the non-encapsulated ribonucleic acid concentration, and C_{All} is the total ribonucleic acid concentration. $EE \left(%\right) = \left(1-{C}_{Free}/{C}_{All}\right) \times 100 %$

For the acid dissociation constant (pKa), a titration method based on 6-p-Toluidino-2-naphthalenesulfonic acid (TNS) was used with a microdisk fluorescence analyzer (Infinite F200 Pro, Tecan, excitation wavelength: 320 nm, emission wavelength: 448 nm) to analyze the fluorescence intensity titration curve with a pH value of 2.5-11.0 and a total of 18 points of the sample in the buffer. The titration curve was sigmoidal fitted, and the calculated acid dissociation constant (pKa) was the pH value when the nucleic acid-lipid nanoparticle carrier reaches half of the maximum fluorescence intensity.

**Table 6**

| | Particle size (nm) | Polydispersity index (PdI) | Zeta potential (mV) | Ribonucleic acid recovery rate (%) | Encapsulation efficiency for ribonucleic acid (%) | Acid dissociation constant (pKa) |
|---|---|---|---|---|---|---|
| Nucleic acid-lipid nanoparticle carrier (1) | 88.5 | 0.33 | -6.16 | 75.8 | 44.8 | NA |
| Nucleic acid-lipid nanoparticle carrier (2) | 92 | 0.18 | -7.35 | 33.9 | 5.1 | 5.02 |
| Nucleic acid-lipid nanoparticle carrier (3) | 66 | 0.15 | -3.44 | 46.3 | 76.2 | 5.36 |
| Nucleic acid-lipid nanoparticle carrier (4) | 63.9 | 0.13 | -11.7 | 52.3 | 81.1 | 5.41 |
| Nucleic acid-lipid nanoparticle carrier (5) | 63.9 | 0.16 | -4.42 | 86.7 | 57.6 | 7 |
| Nucleic acid-lipid nanoparticle carrier (6) | 60.1 | 0.25 | -3.64 | 85 | 59.9 | 7.61 |
| Nucleic acid-lipid nanoparticle carrier (7) | 78.2 | 0.12 | -4.38 | 71.8 | 83.2 | 4.93 |
| Nucleic acid-lipid nanoparticle carrier (8) | 58.4 | 0.14 | -6.93 | 86.6 | 55 | 6.64 |
| Nucleic acid-lipid nanoparticle carrier (9) | 58.6 | 0.11 | -2.42 | 80 | 58.8 | 6.76 |
| Nucleic acid-lipid nanoparticle carrier (10) | 75.1 | 0.07 | -6.33 | 82.4 | 97.2 | 6.45 |
| Nucleic acid-lipid nanoparticle carrier (11) | 99.3 | 0.09 | -4.54 | 61.5 | 94.8 | 6.74 |

Nucleic acid-lipid nanoparticle carriers (12)-(20) obtained in Examples 44-52 were evaluated for particle size, polydispersity index (PdI), Zeta potential, ribonucleic acid recovery rate (mRNA recovery), encapsulation efficiency (EE) for ribonucleic acid and acid dissociation constant (pKa). The results are shown in Table 7.

**Table 7**

| | Particle size(nm) | Polydispersity index (PdI) | Zeta potential (mV) | Ribonucleic acid recovery rate (%) | Encapsulation efficiency for ribonucleic acid (%) | Acid dissociation constant (pKa) |
|---|---|---|---|---|---|---|
| Nucleic acid-lipid nanoparticle carrier (12) | 64.3 | 0.15 | -3.04 | 70 | 55.2 | 5.96 |
| Nucleic acid-lipid nanoparticle carrier (13) | 59.3 | 0.26 | -2.24 | 84.2 | 79.7 | 6.04 |
| Nucleic acid-lipid nanoparticle carrier (14) | 57.9 | 0.19 | -3.19 | 73.1 | 72.8 | 6.06 |
| Nucleic acid-lipid nanoparticle carrier (15) | 138.8 | 0.11 | -5.85 | 63.2 | 44.2 | 7.22 |
| Nucleic acid-lipid nanoparticle carrier (16) | 118.8 | 0.05 | -4.59 | 68.8 | 40.6 | 6.94 |
| Nucleic acid-lipid nanoparticle carrier (17) | 98.1 | 0.08 | -3.65 | 64.7 | 45 | 7.06 |
| Nucleic acid-lipid nanoparticle carrier (18) | 181.2 | 0.14 | -4.02 | 41 | 41 | 7.73 |
| Nucleic acid-lipid nanoparticle carrier (19) | 133.4 | 0.11 | -5.6 | 50.2 | 53.7 | 7.46 |
| Nucleic acid-lipid nanoparticle carrier (20) | 110.9 | 0.15 | -4.85 | 47.3 | 47.2 | 7.47 |

Nucleic acid-lipid nanoparticle carriers (21)-(29) obtained in Examples 53-61 were evaluated for particle size, polydispersity index (PdI), Zeta potential, ribonucleic acid recovery rate (mRNA recovery), encapsulation efficiency (EE) for ribonucleic acid and acid dissociation constant (pKa). The results are shown in Table 8.

**Table 8**

| | Particle size (nm) | Polydispersity index (PdI) | Zeta potential (mV) | Ribonucleic acid recovery rate (%) | Encapsulation efficiency for ribonucleic acid (%) | Acid dissociation constant (pKa) |
|---|---|---|---|---|---|---|
| Nucleic acid-lipid nanoparticle carrier (21) | 147.1 | 0.09 | -5 | 40.9 | 19.7 | NA |
| Nucleic acid-lipid nanoparticle carrier (22) | 129.3 | 0.06 | -5.68 | 54.3 | 37.3 | NA |
| Nucleic acid-lipid nanoparticle carrier (23) | 104.7 | 0.08 | -6.64 | 56.8 | 40.1 | NA |
| Nucleic acid-lipid nanoparticle carrier (24) | 151.3 | 0.1 | -8.32 | 33.5 | 9.2 | 6.82 |
| Nucleic acid-lipid nanoparticle carrier (25) | 123.4 | 0.09 | -5.54 | 64.4 | 55.4 | 6.4 |
| Nucleic acid-lipid nanoparticle carrier (26) | 102.6 | 0.08 | -11.6 | 84.9 | 39.5 | 6.81 |
| Nucleic acid-lipid nanoparticle carrier (27) | 158.1 | 0.11 | -0.23 | 26.5 | 61 | 7.6 |
| Nucleic acid-lipid nanoparticle carrier (28) | 243.6 | 0.16 | 0.79 | 31.3 | 30.6 | 8.12 |
| Nucleic acid-lipid nanoparticle carrier (29) | 194.3 | 0.14 | 0.04 | 20.3 | 38.8 | 7.77 |

Nucleic acid-lipid nanoparticle carriers (30)-(38) obtained in Examples 62-70 were evaluated for particle size, polydispersity index (PdI), Zeta potential, ribonucleic acid recovery rate (mRNA recovery), encapsulation efficiency (EE) for ribonucleic acid and acid dissociation constant (pKa). The results are shown in Table 9.

**Table 9**

| | Particle size (nm) | Polydispersity index (PdI) | Zeta potential (mV) | Ribonucleic acid recovery rate (%) | Encapsulation efficiency for ribonucleic acid (%) | Acid dissociation constant (pKa) |
|---|---|---|---|---|---|---|
| Nucleic acid-lipid nanoparticle carrier (30) | 172 | 0.09 | -4.45 | 41.3 | 11.58 | 7.6 |
| Nucleic acid-lipid nanoparticle carri er (31) | 205.9 | 0.13 | -5.24 | 21 | NA | 8.12 |
| Nucleic acid-lipid nanoparticle carrier (32) | 188.3 | 0.07 | -4.77 | 40.8 | 17.71 | 7.77 |
| Nucleic acid-lipid nanoparticle carrier (33) | 98.3 | 0.08 | -2.87 | 72.7 | 24.9 | 7.55 |
| Nucleic | 120.6 | 0.08 | -6.93 | 77.2 | 30.4 | 7.16 |
| acid-lipid nanoparticle carrier (34) | | | | | | |
| Nucleic acid-lipid nanoparticle carrier (35) | 66.5 | 0.03 | -4.78 | 72.2 | 29.5 | 7.05 |
| Nucleic acid-lipid nanoparticle carrier (36) | 110.7 | 0.07 | -6.41 | 74.9 | 40 | 7.55 |
| Nucleic acid-lipid nanoparticle carrier (37) | 223.6 | 0.16 | -6.49 | 62.7 | 19.9 | 7.57 |
| Nucleic acid-lipid nanoparticle carrier (38) | 97.5 | 0.11 | -4.1 | 74.2 | 41.8 | 7.51 |

Based on Table 6 to Table 9, it is know that the particle sizes of the nucleic acid-lipid nanoparticle carriers formed by self-assembly of the pharmaceutical composition of the present disclosure could be about 50 nm to 250 nm, and the polydispersity index could be between 0.03 and 0.33. In some Examples, the ribonucleic acid recovery rate (%) of the nucleic acid-lipid nanoparticle carriers formed by self-assembly of some of the pharmaceutical compositions of the present disclosure could be higher than about 80%, and the acid dissociation constant (pKa) could range between 5 and 8.5.

### Transfection efficiency and cell viability evaluation

HEK293 cells were seeded into a 96-well culture plate at 5×10⁴ cells/well for adherent culture for 24 hours. Next, 97 µL of Opti-MEM medium and 3 µL of lipofectamine reagent (purchased from Invitrogen) were evenly mixed and reacted at room temperature for 10 minutes to obtain a first solution. 1 µL of luciferase mRNA (product number: R1018, purchased from APExBIO) (concentration: 1 mg/mL) and 99 µL of Opti-MEM^{™} medium were evenly mixed to obtain a second solution. After that the second solution was added to the first solution, mixed evenly, and reacted at room temperature for 5 minutes to obtain a transfection solution. Next, the lipid nanoparticle (LNP) solutions prepared in Examples 35-43 were respectively diluted with 1xPBS to obtain respectively diluted lipid nanoparticle solutions (volume: 200 µL, concentration: 5 µg/mL). Then, 800 µL of cell culture medium (product number: MT-10-009-CVS, purchased from Corning) was added to the respective diluted lipid nanoparticle solutions and mixed with the transfection solution to obtain respective cell co-culture solutions (concentration of luciferase mRNA was 0.1 µg/100 µL). After that, 100 µL of different cell co-culture solutions were respectively added to the 96-well culture plate and co-cultured with the cells (0.1 µg mRNA/well). After 24 hours of culture, 0 µL of supernatant was taken and added to 20 µL of cell viability assay reagent (GF-AFC). After reacting at 37°C for 35-40 minutes, the fluorescence value of the resultant product was measured by a fluorometer (Ex: 380 nm; Em: 540 nm). By dividing the fluorescence detection reading of the sample and the fluorescence value of the untreated negative control group, the cell viability in the presence of different lipid nanoparticle (LNP) solutions was calculated. The results are shown in Table 10.

**Table 10**

| | Cell viability |
|---|---|
| Cell culture medium | **>99%** |
| Nucleic acid-lipid nanoparticle carrier (2) | **>99%** |
| Nucleic acid-lipid nanoparticle carrier (6) | **>99%** |
| Nucleic acid-lipid nanoparticle carrier (7) | **>99%** |
| Nucleic acid-lipid nanoparticle carrier (8) | **>99%** |
| Nucleic acid-lipid nanoparticle carrier (10) | **>99%** |

Based on the foregoing, it is known that the lipid compounds mentioned in the present disclosure hardly affect cell activity when performing *in vitro* cell experimental analysis.

Next, 100 µL of ONE-Glo Reagent was added to each well of the culture plate, and the luminescence value was measured within 3 minutes to evaluate the transfection ability of different lipid nanoparticle (LNP) solutions. The results are shown in Table 11.

**Table 11**

| | Transfection ability | |
|---|---|---|
| Cell culture medium | - | |
| Nucleic acid-lipid nanoparticle carrier (2) | ++ | |
| Nucleic acid-lipid nanoparticle carrier (6) | +++ | |
| Nucleic acid-lipid nanoparticle carrier (7) | +++ | |
| Nucleic acid-lipid nanoparticle carrier (8) | | +++ |
| Nucleic acid-lipid nanoparticle carrier (10) | | +++ |
| | Transfection ability: | |
| | "-" represents: Normalized luminescence intensity (RLU) ≤ 1.2e+03 | |
| | "+" represents: 1.2e+03< Normalized luminescence intensity (RLU) < 1e+05 | |
| | "++" represents: 1e+05≦Normalized luminescence intensity (RLU) < 1e+06 | |
| | "+++" represents: Normalized luminescence intensity (RLU)) ≥ 1e+06 | |

According to Table 11, it is known that the lipid compounds mentioned in the present disclosure can indeed improve the transfection efficiency of HEK293 cells.

It will be apparent to those skilled in the art that various modifications and variations can be made to the disclosed embodiments. It is intended that the specification and examples be considered as exemplary only, with a true scope of the disclosure being indicated by the following claims and their equivalents.

## Claims

1. A lipid compound or a derivative thereof, wherein the lipid compound has a structure represented by Formula (I): 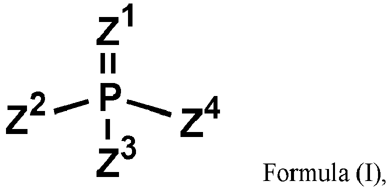
wherein Z¹ is O or S; Z² is -Q¹-A¹-Q²-A²-X¹; Z³ and Z² are the same; Z⁴ is -Q³-A³-Q⁴-A⁴-X²; each of Q¹ and Q³ is independently a single bond, -O- or -NH-; each of Q² and Q⁴ is independently a single bond, -O-, -NH-, -S-S-, 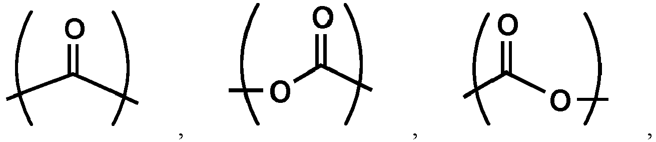 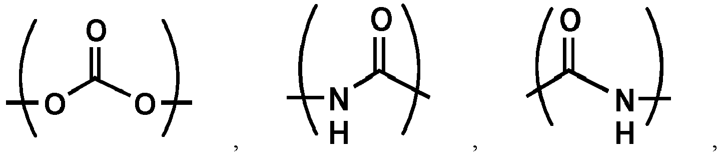 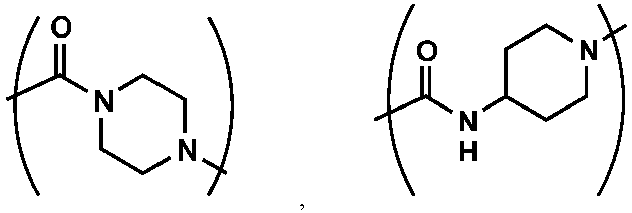 or 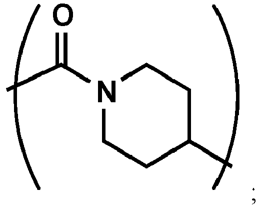 A¹ is C1-C6 alkylene group; A³ is a single bond or a C1-C6 alkylene group; each of A² and A⁴ is independently a single bond, a C1-C12 alkylene group, a C2-C12 alkenylene group or a C2-C12 alkynylene group; X¹ is hydrogen, a C1-C24 alkyl group, a C2-C24 alkenyl group, a C2-C24 alkynyl group, a C1-C24 alkanol group, a C2-C24 alkenol group, a C2-C24 alkynol group, -NR¹R², 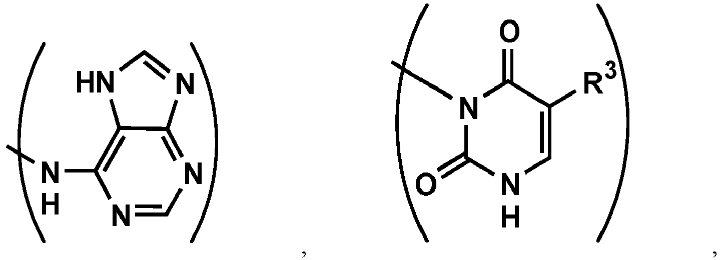 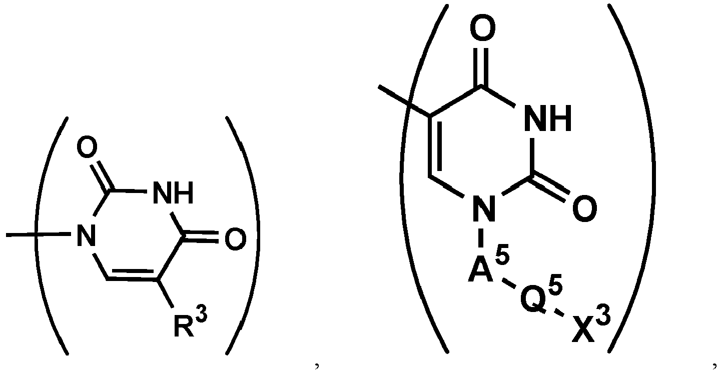 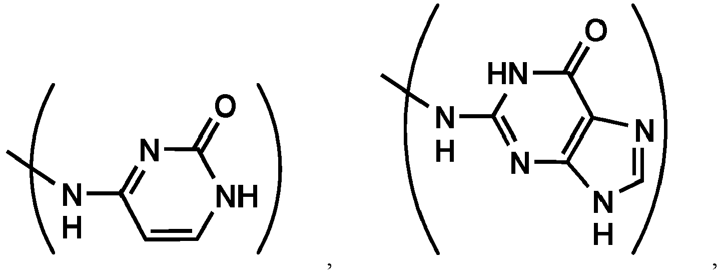 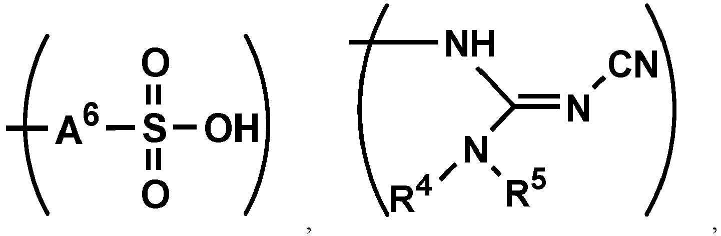 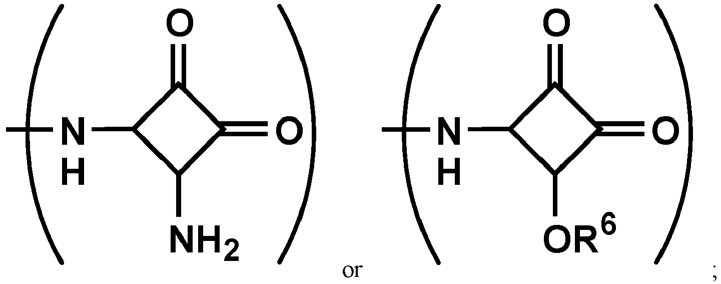 X² is hydrogen, a C6-C12 aryl group, a C1-C24 alkyl group, a C2-C24 alkenyl group, a C2-C24 alkynyl group, a C1-C24 alkanol group, a C2-C24 alkenol group, a C2-C24 alkynol group, -NR⁷R⁸, 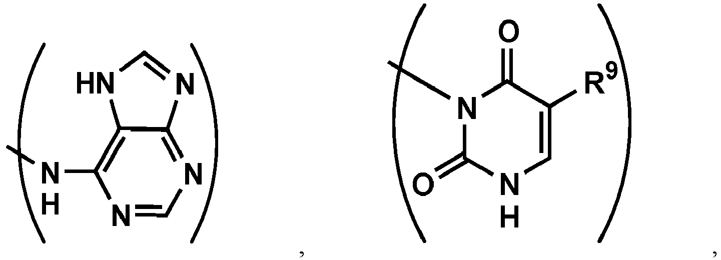 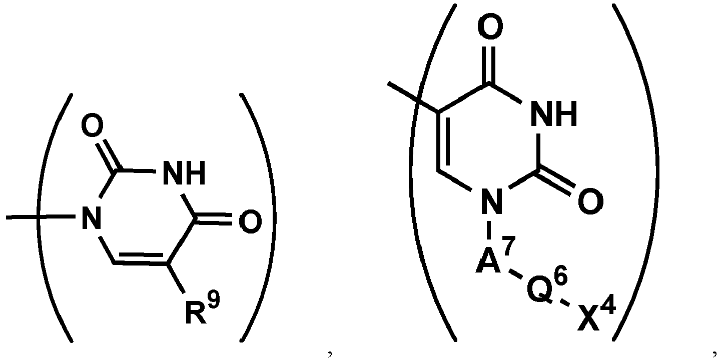 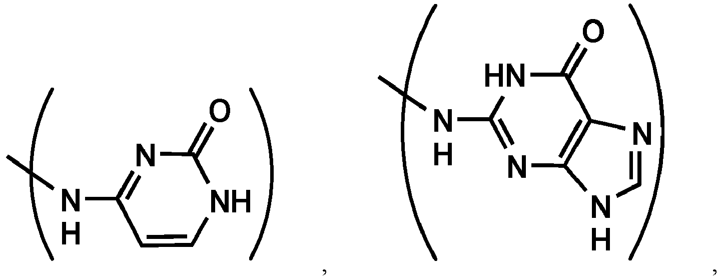 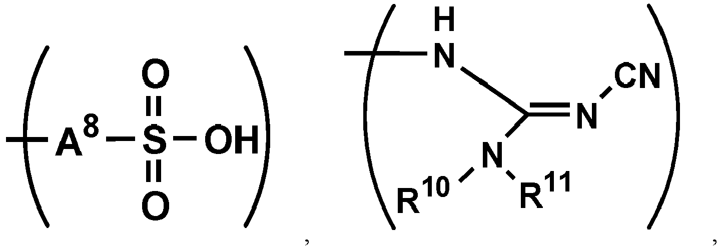 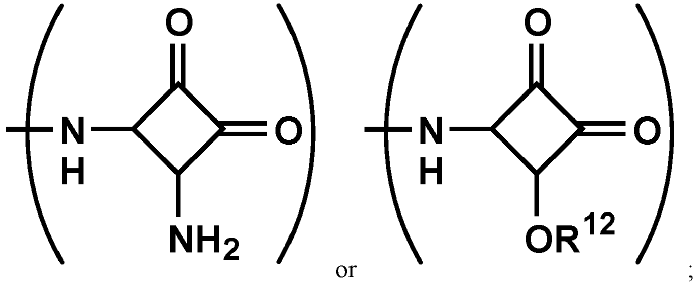
R¹ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A⁹-Q⁷-X⁵; R² is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹⁰-Q⁸-X⁶; each of R³, R⁴, R⁵, R⁶, R⁹, R¹⁰, R¹¹ and R¹² is independently hydrogen or a C1-C6 alkyl group; R⁷ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹¹-Q⁹-X⁷; R⁸ is hydrogen, a C1-C24 alkyl group, a C2-C24 alkenyl group, a C2-C24 alkynyl group, a C1-C24 alkanol group, a C2-C24 alkenol group, a C2-C24 alkynol group or -A¹²-Q¹⁰-X⁸; each of A⁵, A⁶, A⁷, A⁸, A⁹, A¹⁰, A¹¹ and A¹² is independently a C1-C12 alkylene group; each of Q⁵, Q⁶, Q ⁷, Q ⁸, Q ⁹ and Q ¹⁰ is independently -O-, -NH-, -S-S-, 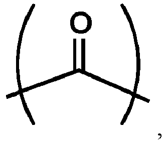 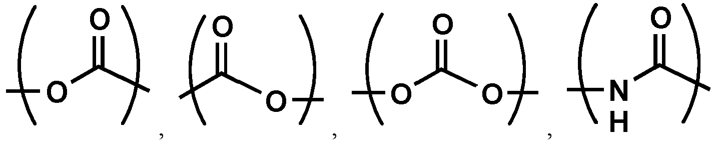 or 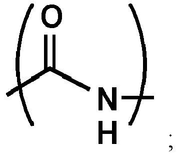 and each of X³, X⁴, X⁵, X⁶, X⁷ and X⁸ is independently a C1-C24 alkyl group.

2. The lipid compound or a derivative thereof as claimed in claim 1, wherein Q¹ is a single bond, and Q² is -O-, -NH-, -S-S-, 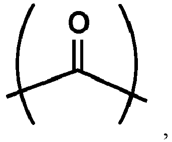 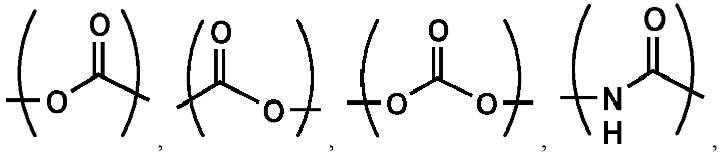 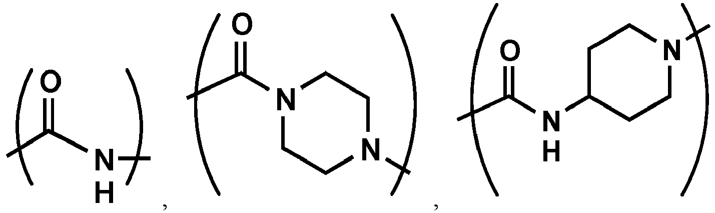 or

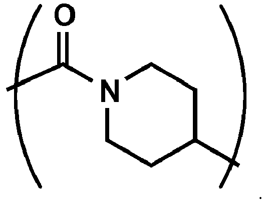

3. The lipid compound or a derivative thereof as claimed in claim 1, wherein Q² is a single bond, and Q¹ is -O- or -NH-.

4. The lipid compound or a derivative thereof as claimed in claim 1, wherein Q³ is a single bond, and Q⁴ is -O-, -NH-, -S-S-, 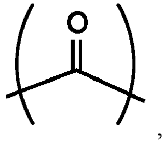 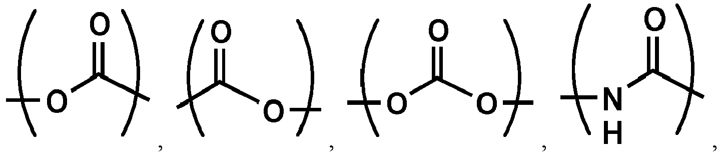 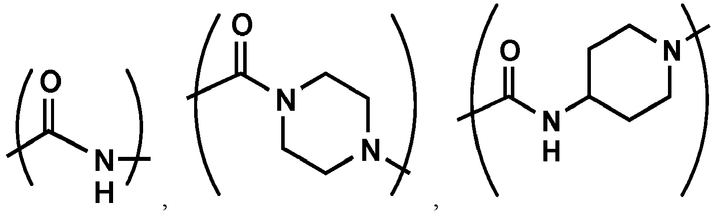 or

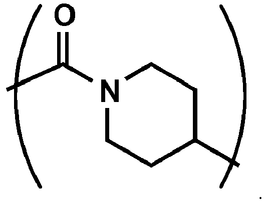

5. The lipid compound or a derivative thereof as claimed in claim 1, wherein Q⁴ is a single bond, and then Q³ is -O- or -NH-.

6. The lipid compound or a derivative thereof as claimed in claim 1, wherein A³ is a single bond, and A⁴ is a C1-C12 alkylene group, a C2-C12 alkenylene group or a C2-C12 alkynylene group.

7. The lipid compound or a derivative thereof as claimed in claim 1, wherein A⁴ is a single bond, and A³ is a C1-C6 alkylene group.

8. The lipid compound or a derivative thereof as claimed in claim 1, wherein A³ is a single bond and A⁴ is a single bond, and then Q⁴ is a single bond and Q³ is -O- or -NH-.

9. The lipid compound or a derivative thereof as claimed in claim 1, wherein Z⁴ and Z² are different.

10. The lipid compound or a derivative thereof as claimed in claim 1, wherein Z², Z³ and Z⁴ are the same.

11. The lipid compound or a derivative thereof as claimed in claim 1, wherein the lipid compound is 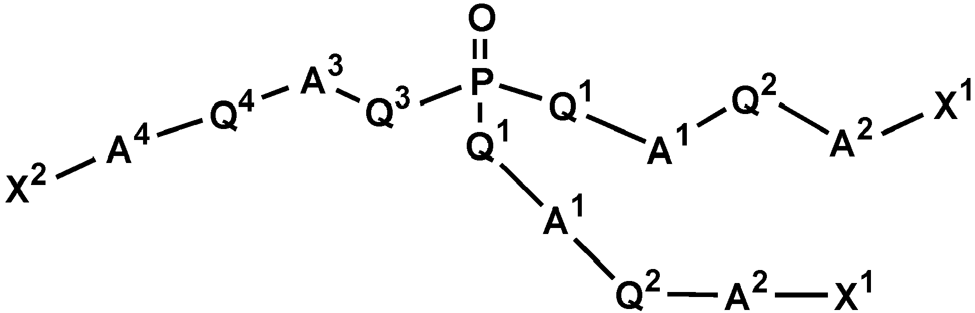 or

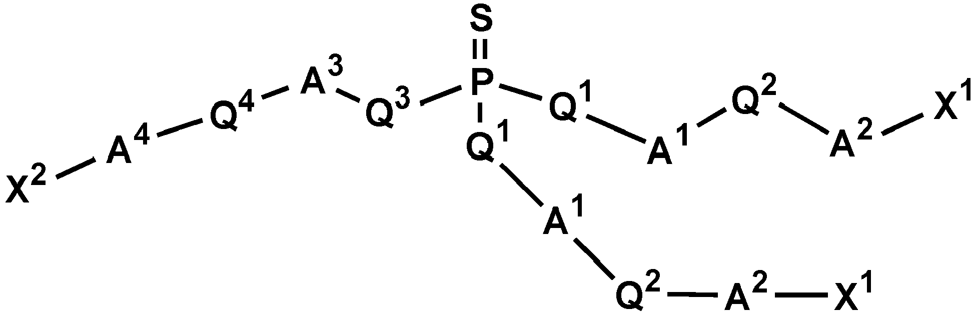

12. The lipid compound or a derivative thereof as claimed in any one of claims 1-11, wherein the derivative of the lipid compound is a pharmaceutically acceptable salt of the lipid compound or a solvate of the lipid compound.

13. A pharmaceutical composition, comprising:
the lipid compound or a derivative thereof as claimed in any one of claims 1-12; and
a sterol.

14. The pharmaceutical composition as claimed in claim 13, wherein the sterol is cholesterol, cholesterol hexasuccinate, ergosterol, lanosterol or a combination thereof.

15. The pharmaceutical composition as claimed in claim 13 or 14, further comprising:
a helper lipid, wherein the helper lipid is 1, 2-dilauroyl-sn-glycero-3-phosphocholine (DLPC), 1, 2-dimyristoyl-sn-glycero-3-phosphocholine (DMPC), 1, 2-dipalmitoyl-sn-glycero-3-phosphocholine (DPPC), 1-palmitoyl-2-stearoyl-sn-glycero-3-phosphocholine (PSPC), 1-palmitoyl 2-oleoyl-sn-glycero-3-phosphatidylcholine (POPC), 1, 2-distearoyl-sn-glycero-3-phosphocholine (DSPC), 1, 2-dioleoyl-sn-glycero-3-phosphocholine (DOPC), hydrogenated soy phosphatidylcholine (HSPC), 1-stearoyl-2-oleoyl-sn-glycero-3-phosphocholine (SOPC), 1, 2-dimyristoyl-sn-glycero-3-phospho-(1'-rac-glycerol)(sodium salt)(DMPG), sulfoquinovosyl diacylglycerol (SQDG), monogalactosyldiacylglycerol (MGDG), digalactosyldiacylglycerol (DGDG), 1, 2-dioleoyl-sn-glycero-3-phosphoethanolamine (DOPE), 1-palmitoyl-2-oleoyl-sn-glycero-3-phosphoethanolamine (POPE), 1, 2-dioleoyl-sn-glycero-3-phospho-1-serine (DOPS), L-a-phosphatidylcholine (EPC), phosphatidylcholine (PC) or a combination thereof.

16. The pharmaceutical composition as claimed in any one of claim 13-15, further comprising:
a polyethylene glycol (PEG)-ylated lipid, wherein the polyethylene glycol (PEG)-ylated lipid is a polyethylene glycol-modified phosphatidylethanolamine, a polyethylene glycol-modified phosphatidic acid, a polyethylene glycol-modified ceramide, a polyethylene glycol-modified dialkyl amine, a polyethylene glycol-modified diacylglycerol, a polyethylene glycol-modified dialkyl glycerol, a polyethylene glycol-modified sterol, a polyethylene glycol-modified phospholipid or a combination thereof.

17. The pharmaceutical composition as claimed in claim 16, wherein the polyethylene glycol (PEG)-ylated lipid is 1, 2-dimyristoyl-sn-glycero-3-phosphoethanolamine-polyethylene glycol (DMPE-PEG), 1, 2-distearoyl-sn-glycero-3-phosphoethanolamine-polyethylene glycol (DSPE-PEG), 1, 2-dimyristoyl-sn-glycero-3-phosphoethanolamine-polyethylene glycol (DMPE-PEG), 1, 2-Dipalmitoyl-sn-glycerol-3-succinate-polyethylene glycol (DPGS-PEG), cholesteryl-polyethylene glycol, 1, 2-dipalmitoyl-sn-glycero-3-phosphoethanolamine-polyethylene glycol (DPPE-PEG), 1, 2-dioleoyl-sn-glycero-3-phosphoethanolamine-polyethylene glycol (DOPE-PEG), 1, 2-dimyristoyl-sn-glycerol-methoxypolyethylene glycol (DMG-PEG), 1, 2-distearoyl-sn-glycerol-3-methoxy-polyethylene glycol (DSG-PEG), 1, 2-dipalmitoyl-sn-glycerol-methoxy-polyethylene glycol (DPG-PEG), α-[2-(ditetradecylamino)-2-oxoethyl]-ω-methoxy-poly(oxy-1, 2-ethanediyl) or a combination thereof.

18. The pharmaceutical composition as claimed in any one of claims 13-17, further comprising:
a nucleic acid, wherein the nucleic acid is deoxyribonucleic acid (DNA), plasmid DNA, messenger ribonucleic acid (mRNA), small interfering ribonucleic acid (siRNA), small activated ribonucleic acid (saRNA), circular ribonucleic acid (circular RNA) or a combination thereof.
